(19) Europäisches Patentamt European Patent Office Office européen des brevets

(11) **EP 4 382 021 A2**

(12) **EUROPEAN PATENT APPLICATION**

(43) Date of publication:
**12.06.2024 Bulletin 2024/24**

(21) Application number: **24172416.0**

(22) Date of filing: **10.02.2020**

(51) International Patent Classification (IPC):
***A61B 1/00*** *(2006.01)*

(52) Cooperative Patent Classification (CPC):
**A61F 9/00736; A61B 1/000094; A61B 1/000095; A61B 1/000096; A61B 34/25; A61B 34/76; A61B 90/20; G06T 7/12;** A61B 90/03; A61B 2017/00115; A61B 2017/00119; A61B 2034/2065; A61B 2090/061; A61B 2090/365; A61B 2090/3735; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2019 US 201962803418 P**

(62) Document number(s) of the earlier application(s) in accordance with Art. 76 EPC:
**20714016.1 / 3 920 858**

(71) Applicant: **THE BOARD OF TRUSTEES OF THE UNIVERSITY OF ILLINOIS**
**Urbana, IL 61801 (US)**

(72) Inventors:
• **LEIDERMAN, Yannek, I.**
**CHICAGO, 60612 (US)**
• **LUCIANO, Cristian, J.**
**CHICAGO, 60612 (US)**

(74) Representative: **Cabinet Laurent & Charras**
**Le Contemporain**
**50 Chemin de la Bruyère**
**69574 Dardilly Cedex (FR)**

Remarks:
This application was filed on 25-04-2024 as a divisional application to the application mentioned under INID code 62.

(54) **IMAGE-GUIDED SURGERY SYSTEM**

(57) An artificial intelligence (AI)-based surgical guidance system configured to provide real-time feedback to a surgeon during a surgical procedure, the system comprising:
a first trained AI model configured to receive real-time images of a surgical field and to identify, in real-time, within the images, one or more tissue boundaries;
an analysis module configured to:
receive, from the first trained AI model, data indicating the one or more tissue boundaries identified by the first trained AI model;
receive data indicating the position of the tip of the surgical instrument;
determine, in real-time, from the data received from the first trained AI model and the data indicating the position of the tip of the surgical instrument, a distance between the tip of the surgical instrument and one of the one or more tissue boundaries;
provide real-time visual, auditory, and/or haptic feedback to the surgeon based on the determined distance between the tip of the surgical instrument and the one of the one or more tissue boundaries.

FIG. 8

**(Cont. next page)**

EP 4 382 021 A2

(52) Cooperative Patent Classification (CPC): (Cont.)
    A61B 2090/502; A61F 9/007; G06T 2207/10021;
    G06T 2207/30041; G06T 2210/41

**Description**

TECHNOLOGICAL FIELD

The present disclosure relates to assistive systems for surgical procedures and, in particular, to image-guided surgical systems that provide real-time feedback to surgeons during a surgical procedure.

BACKGROUND

Surgical procedures include inherent risk to the patient, the mitigation of which is an area of constant research and development. One advance in the field of medical practice has been the move away from open surgery, in which the surgical patient is opened up to expose relatively large areas of the patient's inner cavities, and towards minimally invasive surgeries. Minimally invasive surgeries do not require large incisions, and generally result in faster recovery, less pain, and less risk of complications such as infections.

Many minimally invasive surgeries involve the insertion of one or more surgical instruments through one or more small incisions. Such surgeries include laparoscopic surgeries and robotic surgeries, and generally rely on cameras, microscopes, or other imaging techniques (X-ray, ultrasound, etc.) in order for a surgeon performing the surgery to visualize the surgical field.

However, one difficulty encountered during such procedures is that it can be difficult to accurately understand the visualization of the surgical field provided by medical imaging modalities to the surgeon. For example, diagnostic medical ultrasound provides a two-dimensional image of a space and may show various tissue densities as well as surgical instruments present in the surgical field, but it may nevertheless be difficult for the surgeon to visualize or understand the exact location, in three dimensions, of the surgical instrument(s) relative to various tissues displayed in the surgical field.

In another example, a surgeon may perform an intraocular (i.e., within the eye) surgery using a technique such as optical coherence tomography (OCT), which provides something akin to an "optical ultrasound," by using reflections from within imaged tissue to provide cross-sectional images. Unfortunately, such systems lack needed precision regarding the precise location of the instrument, are adversely affected by imaging artifacts (such as shadows) induced by the material properties of surgical instruments, and do not provide any feedback or guidance to the surgeon or instrumentation. Further, delays in the visual output arise due to computational complexities, so the surgeon may not be visualizing the surgical field in real-time.

While some surgical procedures, particularly microsurgical procedures such as neurosurgery and ocular surgery, have adopted the use of stereo microscopes to allow the surgeon to visualize a surgical field in three dimensions, it can nevertheless be difficult for a surgeon to understand in real-time the relationship between the tip of a surgical instrument and the tissue around that instrument, especially when the surgical field is very small, such as when operating on the eye. Other factors, such as manual precision of instrument control, a dynamic spatial environment resulting from turbulent fluid flow, surgeon experience, and inattentiveness can also cause unintended contact with tissue. This difficulty can increase the risk to the patient, especially when the tissues in the surgical field are particularly susceptible to damage.

For example, during ocular surgery, unintended contact between the surgical instrument and the retina can cause irreversible damage, resulting in serious loss of vision and blindness.

SUMMARY OF THE DISCLOSURE

In accordance with the principles herein, a microsurgical guidance system is set forth. The system includes a haptic device configured to provide direct feedback during a microsurgical procedure from the different sources to a microsurgical instrument in real-time. The system can be configured to receive real-time imaging data from an imaging and/or video capture device, the system configured to analyze and deliver direct modulation feedback concerning a position and/or function of the microsurgical instrumentation. The system can include any suitable image acquisition device, such as an OCT device. The microsurgical guidance system can include a display operatively connected to the haptic device and the microsurgical instrument. The system can further include a feedback loop, whereby the location of the microsurgical instrument in relation to delicate ocular tissues is determined and the effect of instrument-tissue interactions is used in the analysis to guide surgical maneuvers with direct feedback. The feedback loop can be operably connected to both the imaging device and the microsurgical instrument via the haptic device. The feedback loop can be configured to generate an output including a tactile feedback signal, the output can be delivered to the haptic device, and the output can include fine motor feedback, such as graded resistance to movement of the microsurgical instrument approaching a threshold and/or 'no-fly zone' defined as proximity to tissues or structures to be avoided.

The feedback loop can generate an output including a visual feedback signal. The visual feedback signal can include changes to the appearance of the instruments and tissues on the display. The output can include audible feedback to confer information to the surgeon regarding the position of surgical instruments or the effect occurring to the ocular tissues and structures. In this way, precision microsurgical guidance can be delivered via the haptic device by modulating the ratio of force applied to the microsurgical instrument during use in order to achieve a corresponding movement, which can include tremor dampening and fine control of the microsurgical instrument via the feedback.

The direct feedback can be generated based on segmented imaging data defining tissue boundaries and instrument position in real-time. The system can analyze the segmented imaging data via a feedback loop operably connectable to a processor, and generating a feedback output to the system including at least one of tactile, audio and visual feedback.

A method of guiding a microsurgical instrumentation is also set forth. The method includes receiving imaging data in real-time from an imaging system operably connected, directly or indirectly, to the microsurgical instrument. Next, slices or subsections of segmented imaging data can be correlated with instrument position data, if desired, via a processor operably connectable, directly or indirectly, to the microsurgical instrument to produce an output image. The instrument position data can be acquired, at least in part, from a direct haptic feedback device. After the imaging data is analyzed, an output containing feedback is generated in real-time via a feedback loop operably connected to the processor. The feedback to the microsurgical instrument can occur via the direct haptic feedback device and control the power of the microsurgical instrument, if needed. An analysis loop can be configured to distinguish anatomical tissue information from the data and to train the feedback loop via machine learning to discriminate between anatomical layers. Image processing steps can analyze and identify anatomical differences and changes in the segmented imaging data.

In one exemplary embodiment, a surgical instrument controller can be operably connected to the feedback loop. The system can be configured to either generate a warning alert and/or modulate function such as a change in grasping and/or vacuum and/or cutting and/or aspiration and/or application of electromagnetic (laser) or radiofrequency (diathermy) settings of the surgical instrument directly. The display can be configured to receive real-time outputs from the feedback loop, the real-time outputs can be generated by the feedback loop based on instrument position data received from the haptic device, and/or imaging data containing anatomic/structural information received from the suitable imaging device, such as an OCT device. The feedback loop can analyze the instrument position data and the imaging data to produce a visual output and/or an additional haptic feedback output in real-time.

In an exemplary embodiment, a microsurgical guidance system can include an imaging device configured to acquire tissue surround data in real-time. A haptic feedback device can be configured to acquire and send instrument position data derived from a surgical instrument. The feedback loop can be configured to correlate tissue surround data and instrument position data. The feedback loop can be configured to analyze the tissue surround data and instrument position data to form an analyzed data output, and the feedback loop can be configured to generate at least one of a display output, an audible output, and a haptic feedback output based on the analyzed data output. The imaging device can include an image data acquisition device. The image data acquisition device can produce real-time anatomical images. The feedback loop can be configured to merge the real-time anatomical images into a stereoscopic display containing spatial/structural information represented in three dimensions. The feedback loop can be configured to identify tissue planes and/or anatomical structures from of the real-time anatomical images from the imaging device output. The feedback loop can be configured to generate a "no fly zone" from the tissue structure and/or anatomic information and incorporate the "no fly zone" into the output.

In an exemplary embodiment, the visual output and/or the additional haptic feedback output are provided in real-time, and configured to guide users of the system to avoid contacting and/or perturbing defined anatomical structures during a surgical procedure, or to inform tremor dampening, or changing ratio of applied force-to-movement of the surgical instrument mediated by the haptic feedback device in some cases based on feedback force associated with the surgical instrument. The system can be configured to provide feedback for guiding fine movements of a microsurgical instrument that would be more difficult without the feedback, including control based on the power settings of the surgical instrument and fine control of the surgical instrument in preventing removal of tissue, such that the system can be selectively configured to control both the location and the function of the surgical instrument in three dimensions and to facilitate assisted surgical procedures and/or stabilize fine movements of the surgical instrument based on the feedback. The feedback loop can be configured to generate the output within a latency period that allows for modulation of the surgical instruments in real-time.

In one exemplary embodiment, computation time is optimized when the feedback loop is configured to select two orthogonal images, for example, or less than the entire image data file as needed to preserve the improved computational capabilities of the system. In some embodiments a location of the surgical instrument is also analyzed based on a location derived from a suitable source such as the imagining data, haptic device, or other structure or function that provides a coordinate reference system, or the like. The feedback loop can be further configured to process the selected images and compute the distance from a surgical instrument location received from the haptic feedback device all the way down across the selected images to the retina or other tissue of interest in the "no fly zone", considering neighboring pixels.

In some embodiments a stereoscopic or three-dimensional output can be formed based on the imaging data received from the imaging device, wherein the imaging device is a single OCT imaging device.

To further inform the analysis and reduce computation time, additional retina layer or other tissue anatomical information including a loop for informing the feedback loop with distinction parameters between clinically relevant tissue boundaries among anatomical structures, can be provided wherein the loop can be informed regarding the distinction parameters from a learned or stored information database, and/or form additional imaging information obtained from imaging data updates obtained and/or received at an earlier time.

In accordance with the principles herein, in systems configured to guide microsurgical procedures, a method of expediting feedback to the surgeon in real-time can include reducing the volume of the imaged area, if needed; preforming image processing on a selected portion of the imaged area; targeting image processing of tissue boundaries while excluding internal structure (e.g., inner- and outer-retinal boundaries, lens capsule, cornea-aqueous interface) during the image processing; and analyzing the selected portion in view of anatomical changes to the imaged area and microsurgical instrument during the procedure.

BRIEF DESCRIPTION OF THE FIGURES

Fig. 1 is a schematic of an exemplary image-guided surgery system constructed in accordance with the principles herein.

Fig. 2 is a schematic of another exemplary image-guided surgery system.

Fig. 3 is a schematic of yet another exemplary image-guided surgery system.

Fig. 4 shows imaging data information connectable to a feedback loop in an image-guided surgery system.

Fig. 5 shows image output data generated for a display in an image-guided surgery system.

Fig. 6 shows image output data when the instrument enters an exclusion zone.

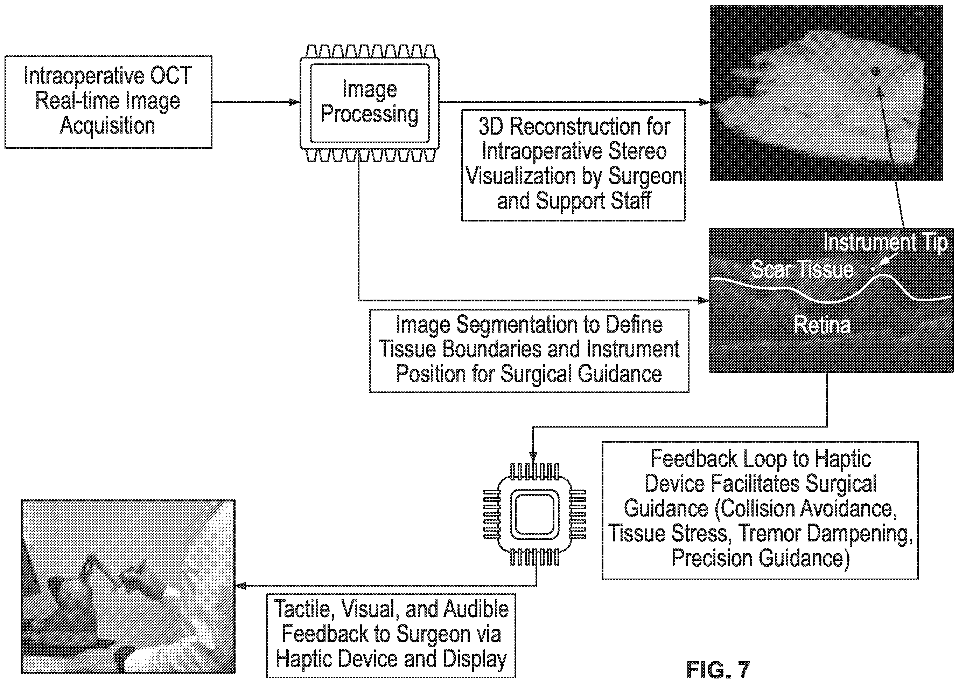

Fig. 7 is a block diagram depicting an example structure for an image-guided surgical system.

Fig. 8 is a block diagram illustrating, in more detail, an example implementation of an image-guided surgical system.

Fig. 9 illustrates a feedback loop formed by components of the image-guided surgical system.

Fig. 10A is an example image received from an imaging system.

Fig. 10B is the example image of Fig. 10A after pre-processing.

Fig. 11 is an manually segmented image depicting the upper and lower boundaries of a retina.

Fig. 12 illustrates an example schema for a convolutional neural network implemented in an embodiment.

Fig. 13 illustrates an embodiment of a UNet schema.

Figs. 14A-14D are an example set of images showing, respectively, an original iOCT image, a ground truth image, a prediction of a trained AI model, and a predicted segmentation superimposed on an input image.

Figs. 15A-15D are another example set of images showing, respectively, an original iOCT image, a ground truth image, a prediction of a trained AI model, and a predicted segmentation superimposed on an input image.

Figs. 16A-16D are yet another example set of images showing, respectively, an original iOCT image, a ground truth image, a prediction of a trained AI model, and a predicted segmentation superimposed on an input image.

Fig. 17 is a graphical depiction of a process for extracting information from images.

Fig. 18 is an example pipeline for analyzing image data received from a stereo image source.

Fig. 19 depicts two images that show an example of Otsu thresholding.

Fig. 20 is an example structure of a CNN for localizing a surgical instrument tip.

Fig. 21 shows an example transformation accomplished using a histogram equalization method.

Fig. 22 depicts two images that show an example of the inputs and outputs of the semi-global block matching algorithm.

Fig. 23 shows an example output of a tip and retina averaging step.

Figs. 24-26 show examples of the output of an example pipline acting on stereoscopic images received from an imaging system.

Figs. 27A-27D depict an example series of images that may be displayed on a display implemented as a feedback device.

Fig. 28 depicts an example method for automatically segmenting and an image and providing feedback in response to a determined tissue boundary.

DETAILED DESCRIPTION

As used herein the term "real-time" refers to the acquisition, processing, and output of images and data that can be used to inform surgical tactics and/or modulate instruments and/or surgical devices during a surgical procedure.

As described above, during various surgical procedures, unintended interactions between surgical instruments and tissue within the surgical field may have unintended consequences, some of which may be permanent. Ophthalmic microsurgery, for example, entails the use of mechanical and motorized instruments to manipulate delicate intraocular tissues. Great care must be afforded to tissue-instrument interactions, as damage to delicate intraocular structures such as the neurosensory retina, optic nerve, lens capsule, and corneal endothelium can result in significant visual morbidity. The surgical guidance system described herein provides a feedback loop whereby the location of a surgical instrument in relation to delicate tissues (e.g., ocular tissues) and the effect of instrument-tissue interactions can be used to guide surgical maneuvers.

Additionally, in embodiments, mechanically induced changes in the volume and position of the tissues (e.g., retina or other tissues) may be used to calculate shear stress and potential tissue trauma to inform and guide surgical maneuvers. Further, other biomarkers assessed via imaging may be used to calculate tissue damage to further inform and guide surgical maneuvers. Feedback to the surgeon can be provided via tactile, visual, and audible cues. Tactile feedback can be conferred via graded resistance to movement of the surgical instrument approaching an exclusion zone defined as proximity to tissues or structures to be avoided. Visual feedback can be conferred via modulation of the appearance of the instruments, tissues, and additional markers on the surgical display, as will be described herein. Audible feedback may serve a similar purpose to visual feedback, to confer information to the surgeon regarding the position of surgical instruments or effect on tissues and structures. Precision surgical guidance may be achieved by modulating the ratio of force applied to the surgical instrument by the surgeon in order to achieve a corresponding movement. In this way tremor dampening and fine control of the surgical instrument may be achieved beyond what is possible via unaided handling of surgical instruments.

System Overview

An image-guided surgical system (IGSS) is described herein. At a general level, the IGSS facilitates the provision of real-time actionable image data and feedback to a surgeon during a surgical procedure. More specifically, the IGSS provides a computer-augmented image and/or other feedback to a surgeon to allow the surgeon to reliably recognize tissue boundaries in the surgical field and understand the relationship between those tissue boundaries and a surgical instrument. Various aspects of the IGSS are described in the following documents, each of which is hereby incorporated by reference herein, in its entirety and for all purposes: G. Aldeghi, Thesis: Retinal Segmentation of Intraoperative B-Scan Optical Coherence Tomography Using Deep Learning, University of Illinois at Chicago; M. De Silvestri, Thesis: Real-time Haptic Guidance System for Retinal Surgery based on Intraoperative Optical Coherence Tomography, University of Illinois at Chicago; M. DiFatta, Thesis: Surgical Instrument Tracking for Intraoperative Vitrectomy Guidance Using Deep Learning and Computer Vision, University of Illinois at Chicago.

An Exemplary IGSS for Ocular Surgery

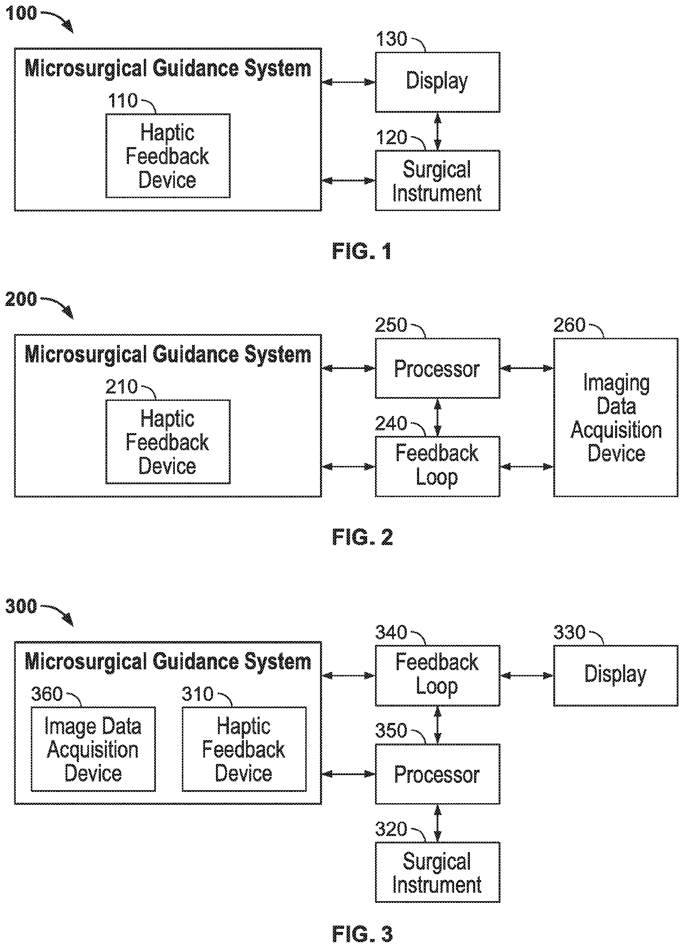

A system constructed in accordance with the principles of the present disclosure, shown generally at 100 in Fig. 1, can include a direct haptic feedback device 110. The direct haptic feedback device 110 can be connectable to a surgical instrument 120 and a suitable display 130. The system 100 can be configured to analyze, generate and send direct visual feedback to the display 130, and direct haptic feedback to the surgical instrument 120 via the direct haptic feedback device in real-time based on both a position of the surgical instrument 120 (as assessed via imaging and the haptic input device) and/or changes occurring in the anatomy of a patient during a surgical procedure.

To this end, in one exemplary embodiment a feedback loop 240 can be operatively connected, directly or indirectly, to a system shown generally at 200 in Fig. 2. Alternatively, the feedback loop 240 can be directly integrated into the system 200. The feedback loop 240 can be configured to provide an operable connection with a processor 250, if desired. In an embodiment, the feedback loop 240 and the processor 250 can be directly integrated into a direct haptic feedback device 210. The feedback loop 240 can be operatively connectable to the direct haptic feedback device 210, and configured to analyze input data from the system 200, generate feedback based on both the input data and/or training data available as an input to the feedback loop 240.

The system 200 can be configured to correlate information regarding the position of a surgical instrument 220 with anatomical information contained in an image derived from a patient during a surgical procedure via a suitable imaging data acquisition device 260. In an embodiment, the system 200 is configured to form an operable connection with the imaging data acquisition device 260.

In other exemplary embodiments a system shown generally at 300 in Fig. 3 can integrate an imaging data acquisition device into components thereof.

In all exemplary embodiments constructed in accordance with the principles of the present disclosure, a system including a direct haptic feedback device creating a feedback loop provides an advantage over known systems. Known systems may display the surgical instrument and the surgical field in real time, but are delayed in displaying, or are not able to display, accurate, real-time quantitative or qualitative data about surgical instrument location or tissue location and movement due to the volumes of imaging data they process when attempting to analyze a surgical instrument location. Further, outputs of known systems lack precision in that they indicate the delayed position of the surgical instrument indirectly with a visual display. Furthermore, where OCT is used to generate the imaging data, OCT is subject to significant imaging artifact arising from the material properties of surgical instruments, i.e., metal and plastic, as clinical OCT imaging devices are optimized for biologic tissue. Unlike the known systems, a direct haptic feedback device can provide surgical instrument location data in accordance with the principles herein. Further, known systems lack a direct feedback mechanism, i.e., a means for modulating the position and/or function of surgical instrumentation.

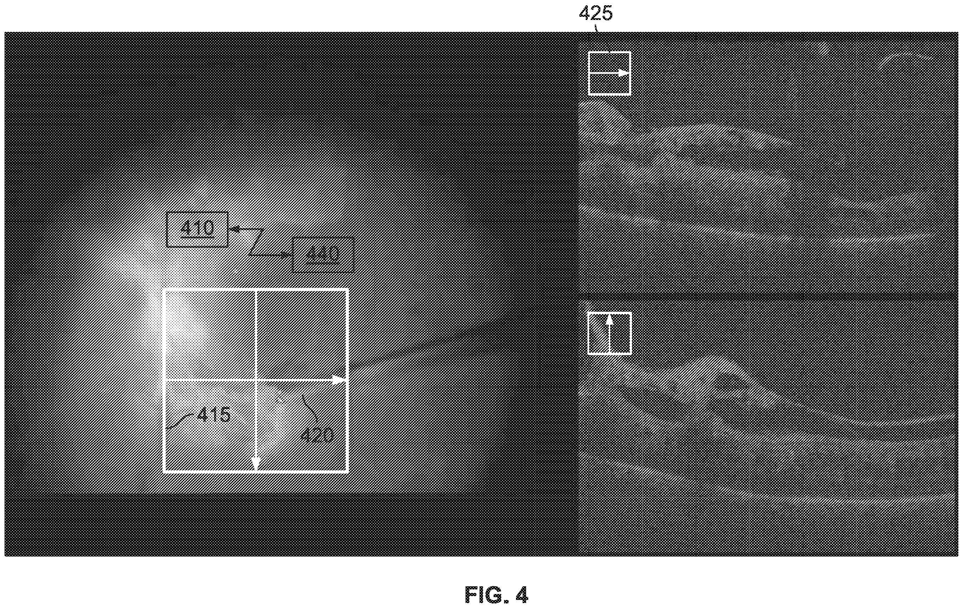

Further, systems constructed in accordance with the principles herein use efficient imaging data analysis to guide the surgeon directly in real-time via both a visual display and a direct haptic feedback device. Certain microsurgical applications involve tissues that are damaged by interaction with surgical instruments and/or in which shear and other forces should be minimized in order to preserve the structure and function of exquisitely delicate biologic tissues. The direct haptic device delivers an increase in resistance to the surgical instrument as the instrument approaches "no fly zone" regions of the anatomy during a surgical procedure. Further, the result of mechanical forces induced by surgical instrumentation on tissues may be assessed directly and in real time via analysis of OCT imaging data, and feedback to instrumentation generated accordingly. For example, in the case of the removal of scar tissue (435 in Fig. 4) from the surface of neural tissue that mediates vision, intraoperative OCT (iOCT) imaging detects deformation of the neural tissue underlying the scar, and if a threshold of tissue deformation is detected, feedback to the haptic device provides resistance to further traction induced by the surgical instrument, preventing damage to delicate structures.

In accordance with the principles herein, real-time images can be analyzed by a feedback loop that segments data received from an image acquisition device into orthogonal slices, for example. For example, using a suitable imaging device such as iOCT, imaging data contained in a cube 415 in Fig. 4 can be resolved into an xy plane slice 425 and an xz plane slice 425' for generating an updated and real-time image of the changing anatomy during the procedure. To this end training data can be incorporated into the image output in addition to the image resolved from the plane slices 425 and 425', respectively. Additionally, in an embodiment a surgical instrument 420 can be precisely located in real-time by inputting location data from a direct haptic feedback device 410 into a feedback loop 440 and correlating the data from the OCT images with the haptic-derived location data. Decreased latency in the interval from imaging to feedback may be achieved via the following mechanisms: reducing the volume of the imaged area, targeted image processing assessing a limited portion of the imaged area, targeted image processing of tissue boundaries while excluding internal structure (e.g., inner- and outer-retinal boundaries, lens capsule, cornea-aqueous interface), image processing a fraction of acquired data (e.g., alternate slices within a plane).

Other improvements that enhance the utility of the surgical instrument in participating in the feedback loop can be incorporated into the system, if desired. For example, atomic force microscopy (AFM) or other sensitized probes for sensitive probing can be incorporated into the system if desired.

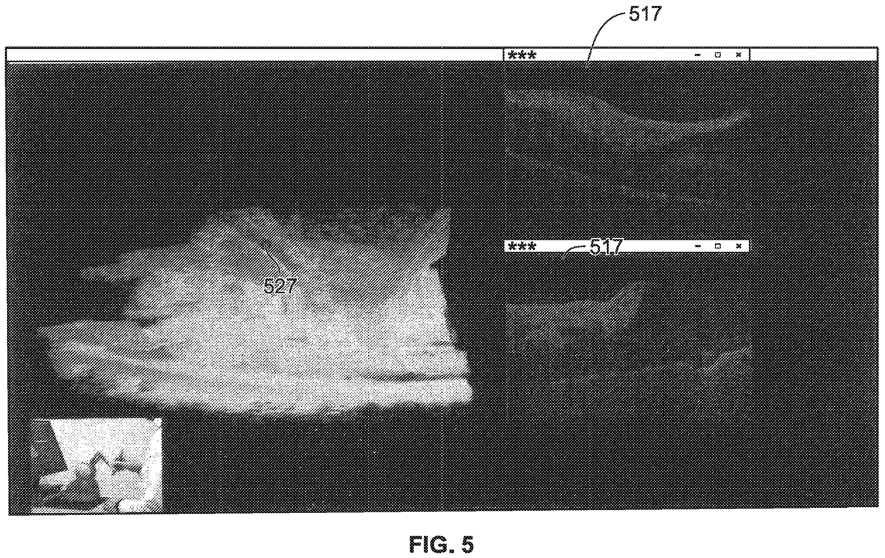

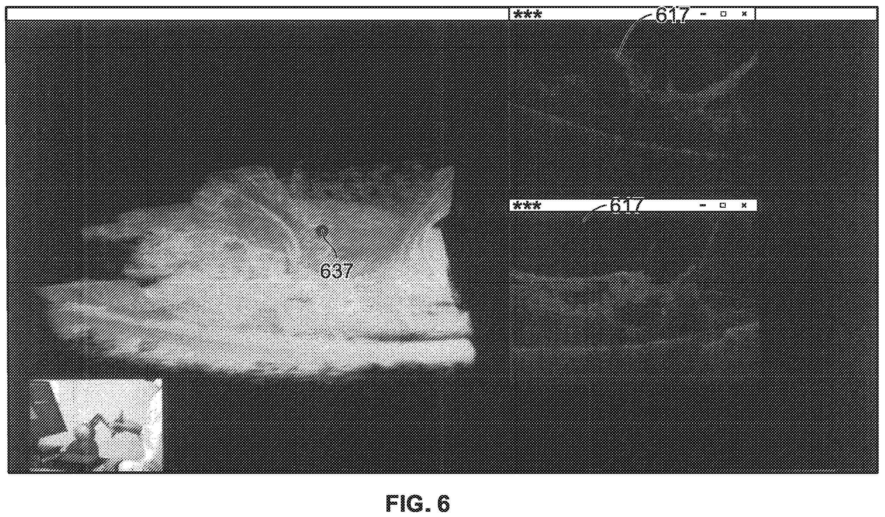

As illustrated in Fig. 5, a line at 517 and 517' comprises a segmentation line defining the inner boundary of the retinal surface. In this figure a dot 527 (displayed in green) contained in the output display image indicates that the tip of the surgical instrument is in a safe zone. When the surgical instrument approaches the retinal surface with a system constructed in accordance with the principles herein, two changes in the system occur happen: first, the color of the dot changes from green to red (Fig. 6), indicating that the surgical instrument is close or proximal to the boundary of biologic tissue that has been defined as an avoidance or exclusion zone, in this case the retina. Alternately the crystalline lens capsule, boundary of the cornea, blood vessels, or other structures may be accordingly defined as boundaries.

Additionally, a direct haptic-mediated force feedback is felt, via the surgical instrument, and the surgeon will feel an increase in resistance to further movement of the surgical instrument, indicating that the instrument is approaching a boundary, as determined by the feedback loop analysis. For surgeons it is very useful to have both visual and direct tactile feedback available in real-time during a surgical procedure that can provide guidance as to the position of the surgical instrument, such as in procedures involving anatomical exclusion zones. Such procedures can include procedures where surgeons must venture in regions of the eye proximal to the retina or other delicate tissues. Further, force feedback mediated by the haptic device is useful during removal of membrane or scar tissue on the surface of the retina using a forceps, aspiration device, vitrectomy probe, or other instrument when a threshold of shear stress or tissue deformation is reached, and feedback prevents exceeding a threshold and resultant tissue damage or loss of function.

Other procedures can be performed in accordance with the principles of the present disclosure, such as cataract removal among others. Additionally systems constructed in accordance with the principles herein can be configured to inform and guide robotic/assistive surgery from a remote location. For example, robotic/assistive surgery can be performed remotely for a soldier located on a battlefield.

Returning now to Fig. 5, a surgeon is free to preform surgical maneuvers here above the retinal surface as indicated in real-time by the (green) dot 527 on the display. However, when the instrument approaches the retinal boundary then the visual display changes in real-time, as is shown in Fig. 6, to display a (red) dot 637 while a direct haptic device delivers resistive force to the surgical instrument simultaneously. As a result, the surgeon has both visual and direct tactile feedback confirming that the tip of the surgical instrument is very close to the retina.

In accordance with the principles of the present disclosure, correlated and analyzed data is generated and displayed in real-time. To this end, any suitable display can be provided. For example, any one or combination of a monitor, headset, microscope, or microcontroller can be provided to display the visual information to the surgeon in real-time. In certain embodiments, suitable displays can include displays configured to display 3D images. In one exemplary embodiment OCT data are processed and reconstructed to display three-dimensional anatomic representations that are offset to accommodate human stereoscopic visual perception, and the images displayed simultaneously using methods for digital three-dimensional viewing. The surgeon user is thus afforded a truly three-dimensional view of the surgical anatomy using OCT data.

Images generated using a feedback loop in accordance with the principles herein can include segmented imaging data, and/or data from a connected haptic device, and/or training data learned by the feedback loop at an earlier time. Image outputs to one or more display can include selected imaging data.

A patient's anatomy changes during surgery. Systems constructed in accordance with the principles herein provide continuous, direct guidance during surgery taking the anatomical changes and incorporating updates therefrom into real-time tactile and visual feedback for the surgeon. Further, guidance for the surgeon from exemplary systems herein can include a direct haptic output to one or more surgical instruments and/or to one or more surgical instrument controllers. For example, the function of surgical instruments can be directly modulated by the feedback loop, such as actuation of forceps or scissors, the cutting and aspiration function of a vitrectomy probe, and the emulsification and aspiration functions of cataract surgical instruments, among others.

Thus, in accordance with the principles of the present disclosure many varied embodiments of systems and devices that are configured to guide surgery are contemplated, with a few exemplary embodiments set forth herein. For example, a device and method for intraoperative Optical Coherence Tomography (iOCT) image-guided microsurgery of the eye are contemplated within the scope of the present disclosure. Specifically, ophthalmic microsurgery is performed using handheld mechanical and motorized instruments in conjunction with an optical surgical microscope that provides magnification of intraocular structures.

Great care must be afforded to tissue-instrument interactions, as damage to delicate intraocular structures such as the neurosensory retina, optic nerve, lens capsule, and corneal endothelium can result in significant visual morbidity. The forces associated with the manipulation of intraocular tissues during surgery are generally below the limit of human tactile force-sensing. The surgeon must use visual cues to assess the magnitude and result of tissue-instrument interactions.

OCT is a widely used imaging modality that confers greater resolution of ocular anatomy than optical viewing relying on magnification of the visible light spectrum. OCT is a non-invasive and safe imaging modality that has recently been integrated into ophthalmic surgical microscopes as an adjunct to optical viewing. At present the surgeon relies on optical viewing in real-time, and OCT data is presented as an adjunct image. While the surgeon may use the ancillary imaging data to inform tactical surgical maneuvers, there is no direct link or feedback loop between the imaging data and instrumentation.

In accordance with the principles herein a microsurgical guidance system that provides feedback to the surgeon and is capable of direct modulation of the position and function of microsurgical instrumentation is set forth. The component structure is as depicted in Figs. 1-3.

Optical and iOCT images can be captured in real-time via digital video capture and OCT devices, respectively. Image processing can be performed and the output utilized for 1) three-dimensional reconstruction of OCT imaging data and graphical display for the surgeon user to allow for execution of surgical tactics and maneuvers in analogy to optical viewing; 2) rapid automated segmentation of OCT and digital images to define anatomic structures and boundaries for collision avoidance and surgical guidance.

Segmentation can be performed using suitable algorithms, such as computer vision or machine-learning algorithms, to facilitate surgical guidance in real-time. Surgical guidance can be achieved via a feedback loop whereby positional data on tissue boundaries and anatomic loci serves as input for a haptic device associated with the surgical instrument. Avoidance of instrument-tissue interactions can be achieved via collision avoidance methodology.

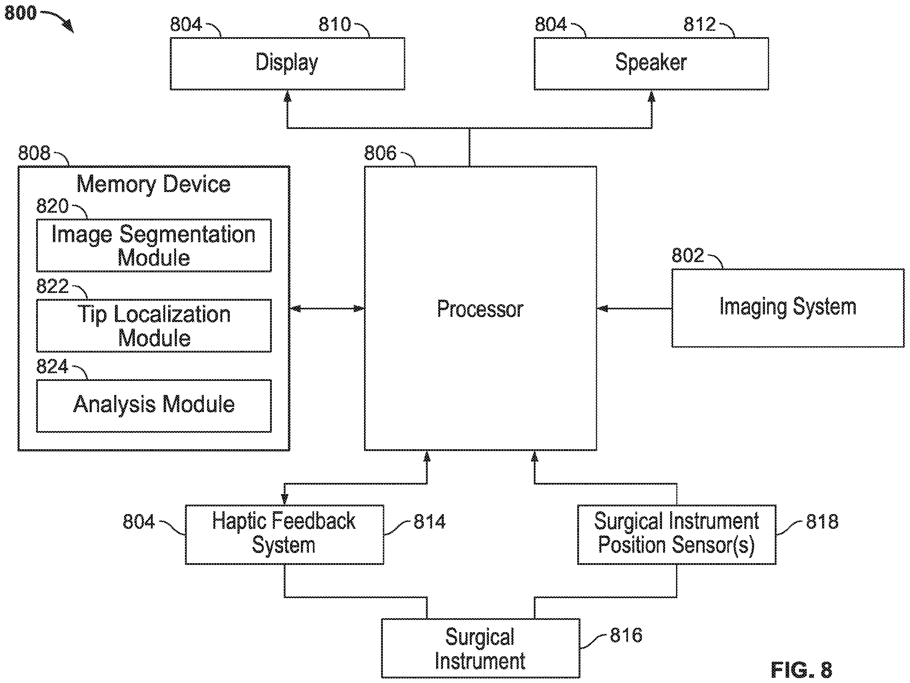

Fig. 8 is a block diagram illustrating, in more detail, an example implementation of an IGSS 800. The example IGSS 800 includes some elements that are optional, as described below, but reflects the general contours of such a system. Generally, the IGSS 800 includes an imaging system 802, one or more feedback devices 804, a computer processor 806, and a memory device 808. The feedback devices 804 may include, in various embodiments, a display 810, a speaker or other noise-generating device (e.g., a piezoelectric buzzer) 812, and/or a haptic feedback system 814. It is contemplated that all IGSS systems would include the display 810, though the display 810 may, in various embodiments, provide more or less feedback to the surgeon, and may provide that feedback in a variety of forms, as described below. It is contemplated, for example, that all embodiments of the IGSS 800 at least provide on the display 810 an image of the surgical field, and that the image is augmented in some fashion to depict, intermittently or continuously, in real-time, within the surgical field, one or more tissue boundaries, which may be identified by software (e.g., an image segmentation module 820) stored on the memory device 808 and executed by the processor 806 to analyze image data received from the imaging system 802.

In various embodiments of the IGSS 800, the display 810 may also include a depiction, in real-time, within the surgical field, of a surgical instrument 816 wielded by the surgeon, and may identify on the display a tip or working end of the surgical instrument 816. The tip or working end of the surgical instrument 816 may be identified by software (e.g., a tip localization module 822) stored on the memory device 808 and executed by the processor 806 to analyze image data received from the imaging system 802 and/or data received from position sensors 818 that sense the position of the surgical instrument 816. The software (e.g., the tip localization module 822) may also, in embodiments, determine a position of the tip of the surgical instrument 816 in three dimensional space within the surgical field.

In embodiments, the IGSS 800 may include an analysis module 824, stored on the memory device 808 and executed by the processor 806, that is operable to determine, in real-time, from the tissue boundary data received from the image segmentation module 820 and from the surgical instrument tip location data received from the tip localization 822, a distance between the tip of the surgical instrument 816 and one of the identified tissue boundaries. In embodiments, the IGSS 800 may, using the determined distance, provide real-time visual, auditory, and/or haptic feedback to the surgeon, via the display 810, the speaker 812, and/or the haptic feedback system 814, respectively.

As will be described below, the imaging system 802 may include an intraoperative optical coherence tomography (iOCT) system in some embodiments, may include a digital stereo microscopy (DSM) system, in other embodiments, may include other surgical imaging systems in still other embodiments, and may, in some embodiments, include multiple types of imaging systems (e.g., may include both an iOCT system and a DSM system).

The display 810 may take the form of one or more of a viewfinder of a DSM system, a monitor, a head-mounted display (such as those used for augmented reality and/or virtual reality systems), and the like.

Generally speaking, in embodiments of the IGSS 800 the processor 806 is bi-directionally communicatively coupled to memory device 808, such that the processor 806 may be programmed to execute the software stored on the memory device 808 and, in particular, the image segmentation module 820, the tip localization module 822 (if implemented), and the analysis module 824, as well as any other software necessary for the operation of the IGSS 800. The processor 806 is similarly communicatively coupled to the imaging system 802, at least receiving image data from the imaging system 802 - though in some embodiments, the processor 806 may be bi-directionally coupled to the imaging system 802 to facilitate providing control functions to the imaging system 802. The processor 806 implements the image segmentation module 820, using as input the image data received from the imaging system 802, and outputs from the image segmentation module 820 data that identifies in the image data tissue boundaries identified in the image data. The identified tissue boundaries, may be added to the image data to provide augmented images for display to the surgeon on the display 810 and/or the identified tissue boundaries may be used to identify and highlight tissue layers (as opposed to merely boundaries) to provide augmented images for display to the surgeon on the display 810.

In embodiments of the IGSS 800 that implement the tip localization module 822, the processor 806 may be programmed to implement the tip localization module 822, using as input to the tip localization module 822 one or both of the image data received from the imaging system 802 and data received from the sensors 818 sensing the position of the surgical instrument 816. The sensors 818 may include sensors that measure the position in space and/or the orientation of the surgical instrument 816 either directly (e.g., through a physical coupling to the surgical instrument 816, such as in the haptic feedback device 814) or indirectly (e.g., by tracking an ultrasonic transducer, by using embedded accelerometers within or attached to the surgical instrument 816, etc.). The tip localization module 822 may output data that identifies and/or localizes in three-dimensional space within the surgical field the tip of the surgical instrument 816. The information output from the tip localization module 822 may be added to the image data to provide augmented images for display to the surgeon on the display 810. Further, in embodiments, the information output from the tip localization module 822 may be used by the analysis module 824 to determine the distance between the tip of the surgical instrument 816 and the identified tissue or tissue boundary, which, in turn, may be used by the processor 806 to provide video, auditory, and/or haptic feedback to the surgeon.

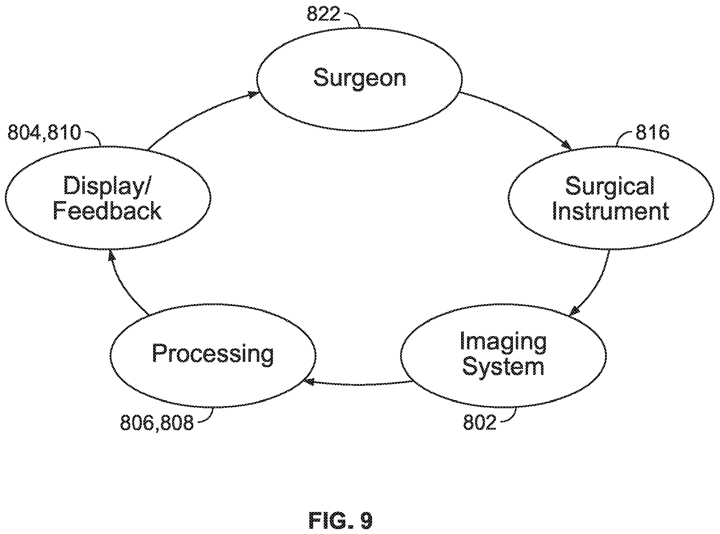

Together the elements of the IGSS 800 form a feedback loop 820, as depicted in Fig. 9. In the feedback loop 820, a surgeon 822 provides input (in the form of direct manipulation or robotic control) to the surgical instrument 818. The surgical instrument 818 appears in the imaging system 802, which provides data to the processing subsystem that comprises the processor 806 and the memory device 808. The processing subsystem provides display data and feedback to the display 810 and the feedback system 804 generally, which is observed by the surgeon 822, who can modify her actions according to the feedback.

As described above, the imaging system 802 may, in some embodiments, particularly those configured to be employed during ocular surgeries such as cataract surgery, vitrectomy, and removal of retinal scar (epiretinal membrane) tissue, include an intraoperative OCT (iOCT) system, a DSM system, or both. The imaging system 802, when configured to be employed during ophthalmic surgeries, may be operative to identify any of a variety of ocular tissues including, retinal tissue, lens tissue, corneal tissue, iris tissue, etc. Each of these systems, and the associated AI models and training methods will be described herein.

Intraoperative Optical Coherence Tomography (iOCT)

An iOCT system is a medical scanner that produces real-time cross-sectional images of the region of interest for a surgical operation. These images allow the surgeon to understand more clearly how various tissues are positioned in the eye. For example, they may allow a surgeon to visualize how scar tissue is positioned over the retina, assisting the surgeon in the important task of avoiding inadvertent significant contact between the surgical instrument(s) and the retina, which could cause irreparable damage to the retina.

The cross-sectional images provided by the iOCT system have a low resolution, which can prevent the surgeon from being able to clearly visualize the boundaries of the imaged tissue. Moreover, the surgical instruments visualized in the iOCT images can interfere with the images by causing shadows and other image artifacts, and may obscure the region of interest to the surgeon.

In the presently described system, image data from the iOCT system (the imaging system 802) may be input into a trained AI model (e.g., a first trained model implementing the image segmentation module 820). The trained AI model may analyze the image data to determine the tissue boundaries and/or layers, so that the data output by the trained AI model indicating the tissue boundaries/layers may be added to the raw image data for display to the surgeon, thereby assisting the surgeon in avoiding contact between a surgical instrument and sensitive tissue.

The trained AI model automatically segments the image data using a deep learning approach. The algorithm consists of a convolutional neural network (CNN) and, in one particular embodiment, employs a UNet architecture (see, *e.g.*, Ronneberger, O, Fischer, P., and Brox, T.: U-net: Convolutional networks for biomedical image segmentation, 2015). The AI model receives as an input the image data obtained from the iOCT imaging system and provides a segmentation probability map of the location of the tissue in question (e.g., the retina, the lens, etc.). The segmentation probability map may also, in embodiments, provide utility measures such as the relative area change and/or volume change of the tissue (e.g., the retina, the lens, etc.) between different images (useful for the surgeon to estimate how much the tissue's area is changing, therefore providing information about the amount of stress the tissue is undergoing at a particular instant in the procedure), the relative change in height of the tissue between images (provides a similar, but different, type of stress indication), the position and/or motion of the tissue relative to adjacent ocular tissues (e.g., to identify retinal detachment), or identify occlusion of an instrument by a tissue, etc. Using the algorithms described herein, the segmentation is achieved at a frame rate of up to or in excess of 58 frames-per-second, allowing the presentation to the surgeon of real-time segmented images presented as augmented raw images.

Image segmentation is a field of computer vision that takes an image, tries to simplify its representation, making it easier to analyze the starting image. Many algorithms exist to produce image segmentation, but most of them work by only analyzing the color of pixels in the image, without any knowledge of what is represented in the images. These approaches are usually the fastest, as no other information other than the pixel value is used to compute the segmentation of the image. While this kind of approach does work quite well in many applications, it does not take advantage of domain knowledge of the images' content. Humans are inherently better at image segmentation tasks. Of course, trying to model all the domain knowledge humans possess is nearly impossible. But some algorithms try to model a specific domain knowledge for a particular segmentation task, aiming to obtain human-like performance. This family of image segmentation algorithms relies heavily on the machine learning concept of convolutional neural network (CNN). These algorithms outperform their counterparts but are costly. To provide better results, the CNN used by these algorithms needs to be trained upon a dataset, in a supervised manner, that includes both the source image and the ground truth segmentation of the image.

The algorithm employed for the iOCT image data to achieve segmentation of retinal tissue in the image data employs a feed-forward neural network (FFNN) that uses supervised learning to segment the retina section within the iOCT images. The CNN employed is composed of three types of layers: convolutional, pooling, and fully-connected, each of which would be understood by a person of skill in the art.

While retina segmentation has been achieved previously using OCT (as opposed to iOCT) image data for diagnostic purposes, because the diagnostic process is not time-sensitive, such segmentation using non-intraoperativeOCT images involves an off-line analysis, which is highly accurate but extremely time consuming for the computer to process. Accordingly, the previously known algorithms employed are not focused on real-time segmentation and cannot achieve interactive rates (on the order of 20-30 frames-per-second) that facilitate real-time provision of image data to a surgeon during a surgical procedure to allow the surgeon to react accordingly. In this way, the processing of iOCT image data for intraoperative surgical guidance, and the presently described algorithms, are novel.

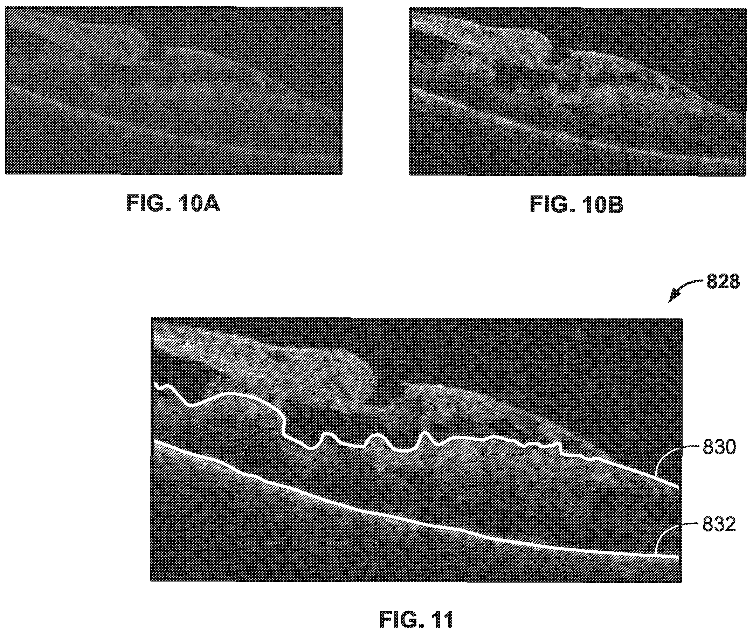

The trained AI model implementing the image segmentation module 820 employed to analyze iOCT data is trained using image data from iOCT systems, which are labelled by experts, as a training set. One such set of unlabeled images is publically available from the American Society of Retina Specialists website, and comprises images that are anonymized. The images are pre-processed in order to help the experts who label the images with the segmentation task. The pre-processing involves applying a fast de-noising function, and increasing the contrast of the images in order to make the elements in the images more easily detectable. The differences between an example initial image and its pre-processed version are depicted in Figs. 10A and 10B, respectively. In embodiments, contrast and brightness may be adjusted, as well, and background noise may be reduced.

For an iOCT image according to the present disclosure, the manual segmentation task used to generate the training set involves annotating the upper and lower boundaries of the retina, as depicted in Fig. 11, using a drawing or annotation tool. In Fig. 11, a training image 828 has been annotated to include a first boundary 830 of the tissue and a second boundary 832 of the tissue. In order to augment the data set, a single, manually segmented image may be used to generate additional segmented images for the training set by flipping, zooming, shifting, and/or rotating the original manually-segmented image to generate derived segmented training images.

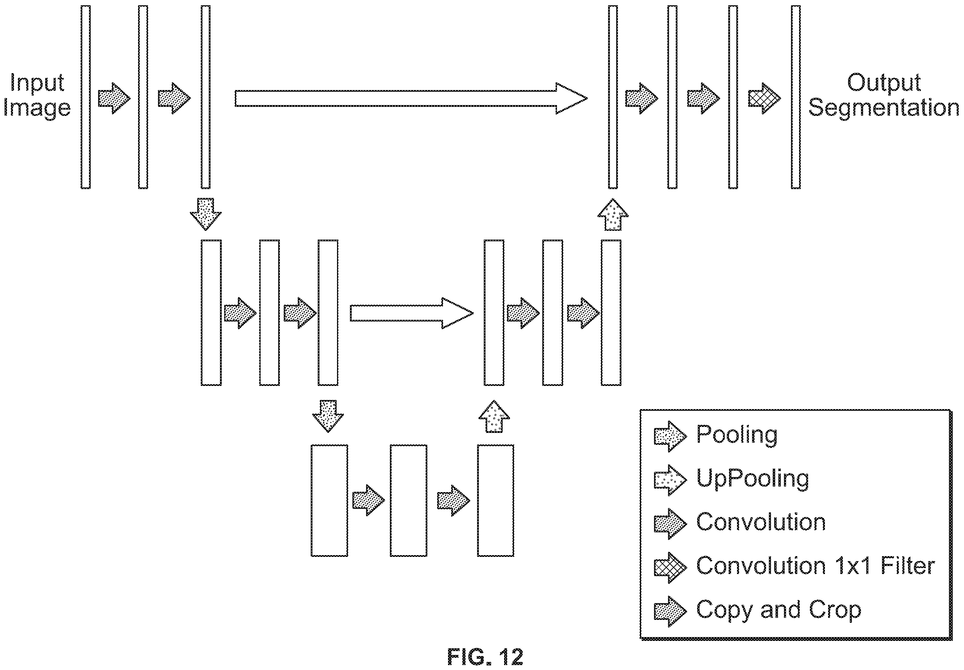

In an embodiment, the deep learning neural network is a particular CNN called UNet, first presented in the paper "U-Net: Convolutional Networks for Biomedical Image Segmentation," cited above. A significant strength of this network is that it obtained astonishing results with small datasets that have been appropriately augmented. A general schema of the UNet is shown in Fig. 12. A UNet is composed of three main parts, that are:

1. Downsampling: The input image is processed by two convolutional layers before it is downsampled with a usual pooling layer. The more the images are downsampled the richer the representation of the data becomes.

2. Bottleneck: This part of the process begins right after the last pooling layer. The data representation here is the richest.

3. Upsampling: Once the bottleneck has elaborated on the data, the data need to be reshaped in order to obtain the wanted output size. This is done by a series of upsampling layers and two convolutional layers. The upsampling layer can be seen as the opposite operation of the pooling layer. The result of the upsampling layer is concatenated with the output of the convolutional layer at the same depth in the downsampling part. When the desired output size is reached, a last 1x1 convolution is applied in order to obtain the segmentation probability for each pixel. Therefore, to obtain an output probability particular activation functions bounded between 0 and 1 need to be used, such as the sigmoid or softmax functions.

The reason behind these skipped connections is because the locality features are lost during downsampling. Therefore, the information flow from the upsampling layers is combined with previously extracted locality features, allowing a more precise output.

While some iOCT images are rendered in the RGB color space, by transforming such images from the RGB color space into a grayscale color space, the number of bytes for each pixel is reduced from three to one, drastically reducing the memory requirements and, at the same time, the processing power required to process the images, and increasing the speed at which images can be processed, both during the training phase of the AI model and during the execution/application of the AI model to real-time data.

In an embodiment, the UNet presents five downsampling and upsampling blocks. Each block is composed of two consequent sub-blocks. Between the two blocks, a dropout layer is implemented. A dropout layer is one in which during the learning procedure neurons are randomly disconnected, and therefore they do not contribute to the output. The number of neurons disconnected is a hyperparameter that can be tuned. Each of the sub-blocks includes: (1) a convolutional layer composed of n filters, each filter with a size of 3x3, and a batch normalization layer employing batch normalization as a technique for improving the speed, performance, and stability of the neural network.

The rectified linear unit (ReLU) activation function is now discussed. The first downsampling block possesses 32 filters, the second 64, and so on until the last downsampling possesses 512 filters. Every pooling layer used for the downsampling uses a 2x2 filter with strides 2. The bottleneck is composed as one of the previously discussed blocks, with two subsequent convolutional layers, where each of them contains 1024 $3\times3$ filters. The upsampling blocks work in the same manner, where the upsampling block possesses the same number of filters as the downsampling block at the same depth.

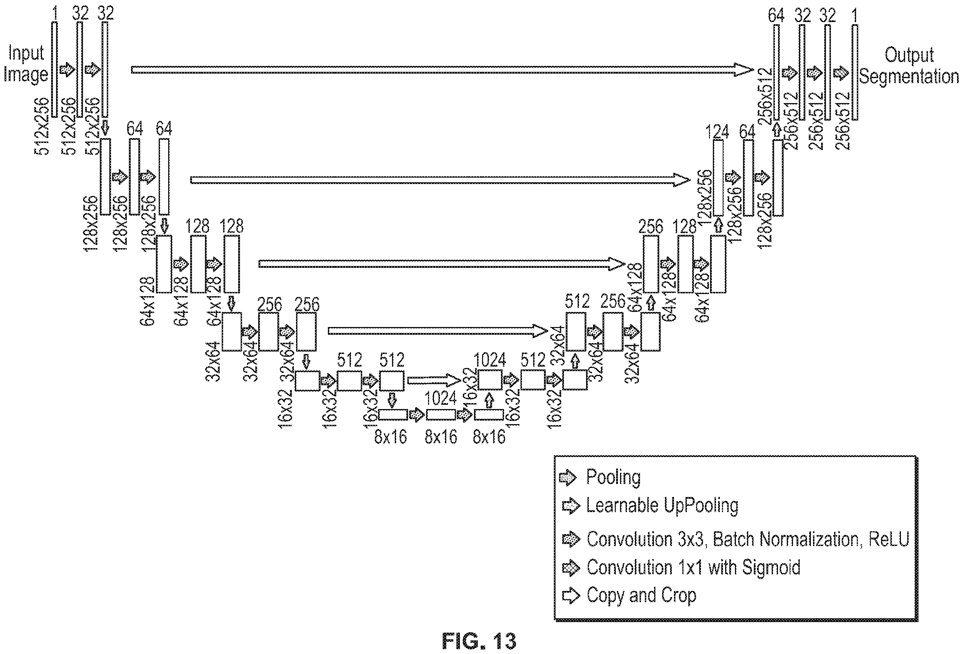

One peculiarity in the embodiment is how the data are upsampled: in fact, the embodiment does not implement an "inverse" of the pooling layer, but a more complex element. The upsampling procedure is executed by a convolutional layer, which therefore during the training will learn the best way to enlarge the data. In the last layer, a sigmoid activation function is used as we need to assign a probability to each pixel of the starting image. In this way, a prediction map is obtained that will be evaluated and processed in order to obtain the desired retina segmentation. An embodiment of the UNet schema is depicted in Fig. 13.

The various hyperparameters of the UNet schema include training batch size (i.e., the number of images in the training batch), the type of optimizer (e.g., stochastic gradient descent (SGD) or Adam, spanning learning rates of 0.001 to 0.01), the loss type (e.g., Dice, Focal, or a balanced linear combination of the two), dropout (e.g., the amount of neurons to drop, ranging from 0% to 25%), early stopping (what epoch sizes are allowed for searching the best model), initial number of filters, and whether to employ batch normalization. In an embodiment, the selected hyperparameters are: SGD as the optimizer, a batch size of 50 images, a learning rate of 0.003, a dropout of 10%, an early stopping size of 20, and 32 initial filters.

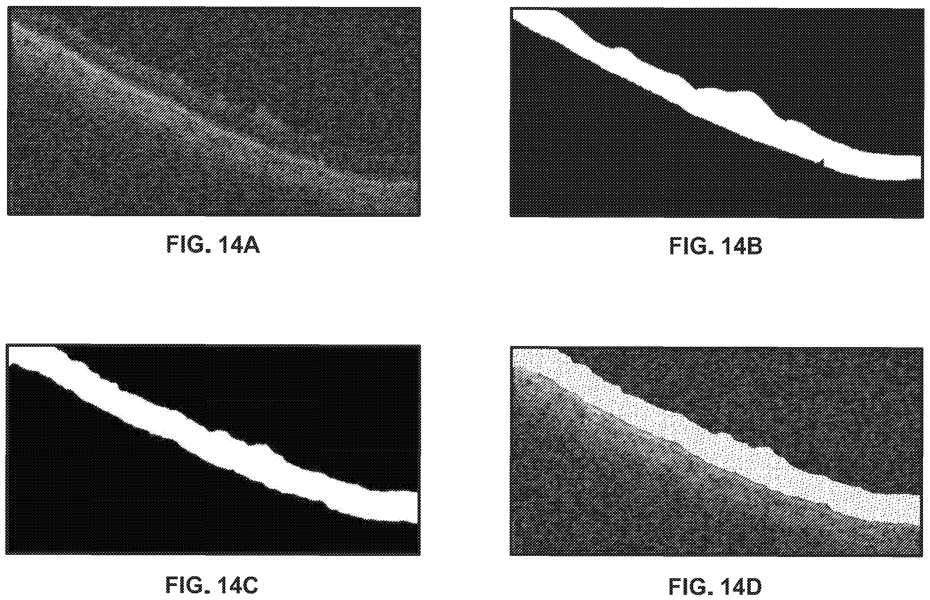

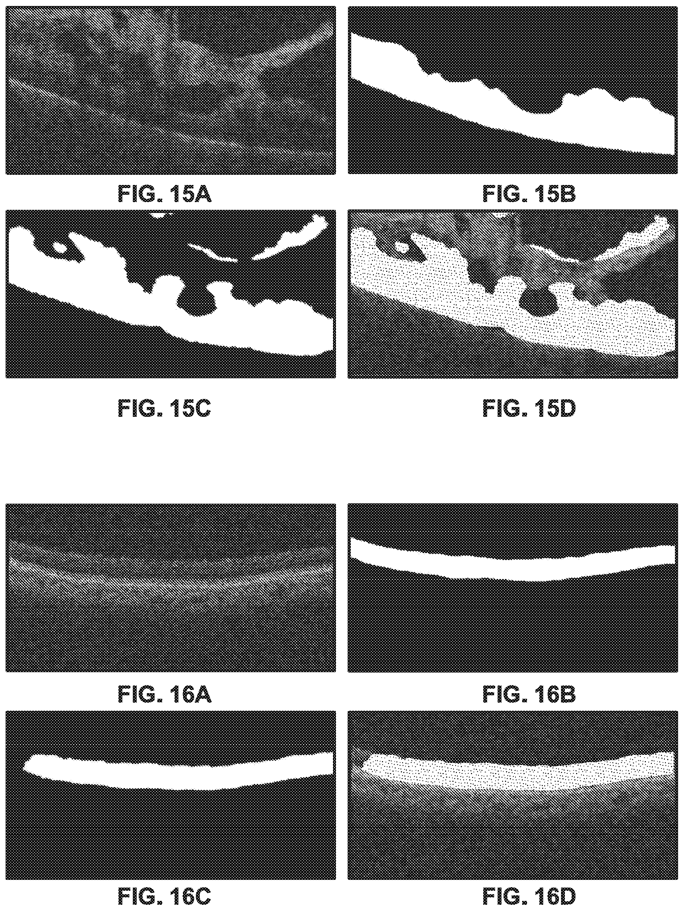

The resulting trained AI model employed for segmenting iOCT images of retinal tissue exhibits excellent generalization ability, often exceeding in degree of accuracy the expert that created the training data. Figs. 14A-14D, 15A-15D, and 16A-16D, are three example sets of images. In each set of images, image "A" represents the original iOCT image, image "B" shows the ground truth (i.e., the actual layer as it would be corrected segmented), image "C" shows the prediction of the trained AI model, and image "D" shows the predicted segmentation superimposed on the input image, as would be displayed to a surgeon in embodiments of the contemplated IGSS 100.

As should be understood, in embodiments in which a volumetric reconstruction of the surgical field (or a portion thereof) is desired or required, a three-dimensional representation of the segmented images may be achieved by combining and analyzing a series of adjacent cross-sectional images. Such embodiments would enable visualization of the instrument and its tip relative to the imaged tissue in three dimensions, thereby providing additional information to the surgeon, and allowing for avoidance of inadvertent contact between the instrument tip and the tissue in the x-, y-, and z-dimensions of the surgical field.

In some embodiments, the IGSS 100 need not identify the tip of the surgical instrument because the tip of the surgical instrument will always be centered in the image data provided by the imaging system 802 as a result of the relative positioning of an imaging device of the imaging system 802 and the surgical instrument 816. For example, the imaging device of the imaging system 802 may be physically coupled to the surgical instrument 816 in a manner that ensures that the field of view of the imaging device is always centered on the tip of the surgical instrument 816. In such embodiments, the distance between a tip of the surgical instrument 816 and a tissue boundary may then be calculated based on a distance between a known pixel location of the tip of the surgical instrument 816 and the identified boundary of the tissue.

In other embodiments, the tip of the surgical instrument 816 may be identified using computer vision techniques and/or a trained AI model, as described herein with respect to determining tissue boundaries. For example, in embodiments, a trained AI model may implement tip localization module 822. The trained AI model may be trained according to principles similar to those used to train the image segmentation module 820 when it is implemented as an AI model. That is, the model may be trained using a series of images of instrument tips present in the surgical field, in which images the tip of the surgical instrument has been manually marked/identified.

In another example embodiment, image processing and segmentation module 820 may implement a computer vision library, such as the Open Source Computer Vision Library (OpenCV), available under open-source license and freely available. In such embodiments, image segmentation module 820 is implemented not as a trained AI model, but rather using computer vision techniques that do not require the training of the model or the attendant manual segmentation required as a training input for the model. The classes and methods of OpenCV provide powerful and simple tools to deal with basic and complex computer vision related issues including advanced image processing, machine learning, object recognition, data visualization, and the like.

cv::MAT is a basic class in OpenCV for dealing with 2D images, such as the iOCT cross-sectional images received from the imaging system when implemented as an iOCT system. Both single and multi-channel images can be loaded as n x m matrices of any data type, and specific members and methods allow access to any information about the image (e.g., size, number of channels, etc.) as well as performance of basic operations (e.g., cropping, copying, resizing, etc.). Each element of the matrix can be easily accessed and managed exploiting the cv::Point class. Each cv::Point object is basically an indexed element $p(i,j)$ with

$$i : i \in \mathbb{N} \ \wedge i < n$$

and

$$j : j \in \mathbb{N} \ \wedge j < m$$

where $n$ and $m$ are respectively the numbers of rows and columns of the image. The methods cv::imread() and cv::imwrite() can be used to load an save images in any of the most common formats, and cv::imshow() provides an easy way to display any loaded image. Built-in filters are also provided, including median, Gaussian, and moving average filters, Sobel edge detection, and many thresholding filters. Each of the filtering tool has a different set of parameters, allowing it to be fined tuned to reach a desired result.

In embodiments implementing OpenCV, a computationally efficient and effective edge detection algorithm may be defined. In embodiments, the core functionality (i.e., segmentation of iOCT images) may be implemented using a well-known segmentation algorithm called Watershed. (*See, e.g.,* Vincent, L. and Soille, P.: Watersheds in digital spaces: an efficient algorithm based on immersion simulations. IEEE Transactions on Pattern Analysis & Machine Intelligence, (6):583-598, 1991; Beucher, S.: Use of watersheds in contour detection. In Proceedings of the International Workshop on Image Processing. CCETT, 1979; Meyer, F. and Beucher, S.: Morphological segmentation. Journal of visual communication and image representation, 1(1):21-46, 1990.) As described above, by analyzing a set of images representing a volume, and stacking the images one on top of the other, a volume may be reconstructed. If an images is considered a two-dimensional matrix, a volume can be an equivalent three-dimensional matrix, the sections of which are the original images. Each pixel is thus extruded into a voxel (a volume pixel), filling the space between consecutive slices, with the resolution of the obtained volume depending on the distance between the successive images, as well as on the lateral resolution of the images.

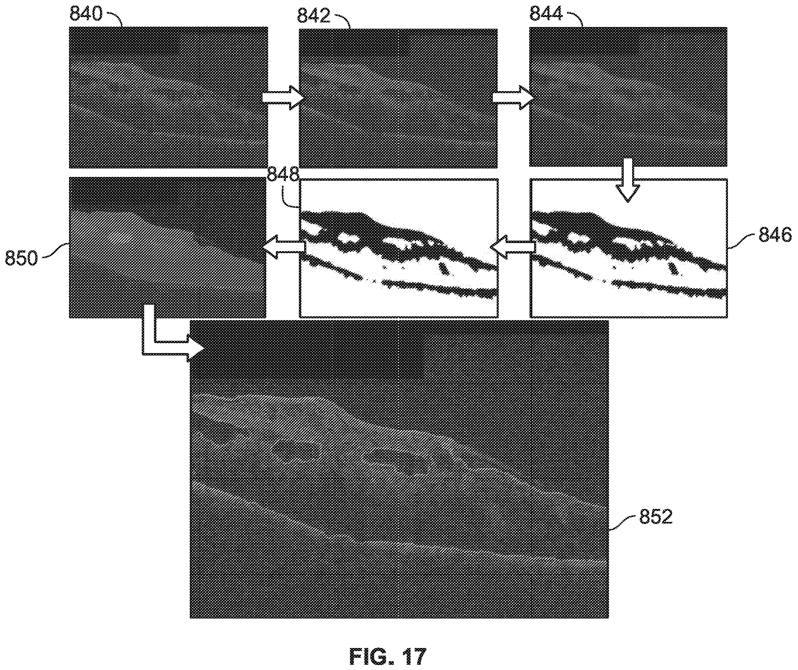

In an embodiment depicted graphically in Fig. 17, a process designed to extracted information from the images includes averaging, filtering, cropping, marker identification, and segmentation.

The transition from an image 840 to an image 842 in Fig. 17 represents the averaging step. It is reasonable to assume that the noise in any of the analyzed images is stochastic in nature. Therefore, using the whole set of images it would be possible reduce the effect of this noise by averaging the images by averaging consecutively acquired images to result in a less noisy image and compensate for spacing between images, thereby creating smoother transitions between successive slices.

The transition from the image 842 to an image 844 in Fig. 17 represents the filtering step. Images may be generally corrupted by high frequency noise, which significantly affects the identification of edges. Given the impulsive nature of the noise, a median filter is considered the best solution. A squared kernel is swiped across the image, and the central pixel is replaced with the median value of all of the pixels inside the kernel. While an average filter, as opposed to the preferred median filter, is usually a good alternative for de-noising images because it works perfectly with stochastic noise, it is ineffective with impulsive noise. There are different kernel sizes that may be used for the filtering step, but it must be considered that, according to the embodiments herein, the parameter has a double effect: on the one hand, a bigger kernel improves de-noising, while on the other hand, a bigger kernel results in a significant increase in computational demand. Accordingly, these two factors must be taken into account when designing the system.

Cropping the images is a simple solution to have a sufficiently big kernel while keeping short the overall processing time. Considering that the surgically relevant area is the area around the surgical instrument(s), there is no need to scan the entire section of the images. As a result, the images may be cropped to an area centered on the tip of the surgical instrument, once identified (as described below). However, this must be balanced by the sensitivity of the segmentation algorithm to the size of the image.

The transitions from the image 844 to an image 846, and from the image 846 to an image 848, in Fig. 17 represents the markers identification step. This is a fundamental step of the algorithm, owing to the nature of the segmentation algorithm when Watershed is used for segmentation. Markers are roughly segmented regions selected, *a priori,* and necessary in order to attempt a finer segmentation only in interesting zones of the images/volume. The first step is binarization via thresholding (image 844 to image 846). Thresholding separates the foreground and background of the images. In an embodiment, a thresholding method called Otsu's method (see, e.g., Zhu, N., Wang, G., Yang, G., and Dai, W.: A fast 2d otsu thresholding algorithm based on improved histogram. In Pattern Recognition, 2009. CCPR 2009. Chinese Conference on, pages 1-5. IEEE, 2009) is employed. Briefly, Otsu's method includes selecting the optical threshold by minimizing the inter-class variance between the two sets of colors (black and white). Additionally, in embodiments, inversion of the colors of the binarized image may result in better thresholding results.

After thresholding, morphological opening is applied (image 846 to image 848). This sub-estimates the black area, resulting in markers that will represent all of the remaining black regions.

All of the steps previously described with respect to Fig. 17 are necessary to achieve segmentation using the watershed algorithm, in which some regions of the image are identified as wells. In the present implementation, the markers identified previously are the wells (or basins) because the method considers the gray scale image as a three-dimensional landscape, where white is high ground, and black is a basin. This "landscape" is progressive filled with "water," as if it were flooded (represented in the transition from image 848 to image 850 in Fig. 17). As the level of the water increases, some of the basins will merge, resulting in identification of a boundary between two adjacent regions. The algorithm performs contours extraction (the transition from image 850 to image 852 in Fig. 17), finding all of the dims separating the basins, and stores them as an array of pixels belonging to the original image.

The watershed algorithm is a simple albeit powerful tool, but its efficacy relies entirely on the processing phase that precedes it. In particular, this algorithm is known to be sensitive to the problem of over-segmentation. An accurate selection of the markers is the only way to prevent it. The reason that cropping thin columns to process might prejudice the correct segmentation is related to this issue. In particular for binarization, a broad view of the whole image results in a more effective threshold selection, being the threshold selected depending on the image histogram. A trade-off thickness of 30 pixels was selected as column thickness, in an embodiment.

The method just explained can be used to identify and separate the main tissue layers in the iOCT images. To accomplish the real aim of the work, though, an additional step is needed. The development of an autonomous guidance system requires the ability to discriminate between the different tissue types segmented in the image. The solution to this problem in the ocular realm was based on the knowledge of the dataset, but it could reasonably be applied to more general cases. The number of interfaces (edges) identified tell us something about how many different tissues we are encountering, and this is enough to know what those tissues comprise, because the positioning of the tissues relative to one another is always the same. The images contain at most three portions. The farthest one from the tool is always the bottom layer of the retina, which can easily be used as a reference. When we are facing three interfaces, one is the bottom of the retina and the other two are necessarily the external surface of the retina and the epiretinal membrane. When only two interfaces are spotted, either there is no membrane or the membrane and the retina are at close contact.

Similar algorithms may be used to implement tip localization in embodiments in which the imaging system 802 includes an iOCT imaging system. That is, in embodiments, a CNN may be trained and deployed as the tip localization module 822. The CNN may be trained using iOCT image data that have been manually annotated to indicate the presence and position in the image data of a tip of a surgical instrument and, thereafter, the trained CNN may accurately identify the presence and position within an image received from an iOCT imaging system of a tip of the surgical instrument, and may determine a distance between the identified tip of the surgical instrument and the boundary of the identified tissue (e.g., the analysis module 824 may compare the identified coordinates of the tip of the surgical instrument to the coordinates of the tissue boundary). Similarly, computer vision techniques may be implemented to reliably identify the tip of the surgical instrument and its position in images received from iOCT imaging system, and the analysis module 824 may compare the coordinates of the tip of the surgical instrument to the coordinates of the tissue boundary.

Digital Stereo Microscopy

Surgical microscopes are currently employed in the operating theater to provide a magnified view of various surgical fields. In particular, surgical microscopes employed during ophthalmic surgeries provide a clear, magnified view of the eye, particularly, for example, the anterior and posterior segments of the eye. Of course, two-dimensional images produced by single-view surgical microscopes eliminate any perception of depth that the surgeon might hope to have. Recently, binocular optical microscopes have been supplanted by digital stereoscopic viewing systems that allow for three-dimensional perception of the surgical field using sterocameras and digital displays.

The contemplated embodiments employing digital stereo microscopy are described with respect to an ophthalmic procedure and, in particular, with respect to an example procedure in which the methods and system are employed to provide reliable information about the distance of a surgical instrument's tip from the surface of a retina during a vitrectomy procedure. However, it should be understood that these methods may be generalized by those of skill in the art to apply to other procedures performed on other tissues.

In the example procedure, the contemplated embodiments determine XY coordinates of a surgical instrument's tip in two-dimensional space, estimate the depth of the tip (i.e., the Z coordinate) to locate it in three-dimensional space, and perform computation in real-time to provide a surgeon with the position of the surgical instrument's tip in three-dimensional space. In embodiments, these data may also be used, as described throughout this specification, to provide visual, auditory, and/or haptic feedback to the surgeon.

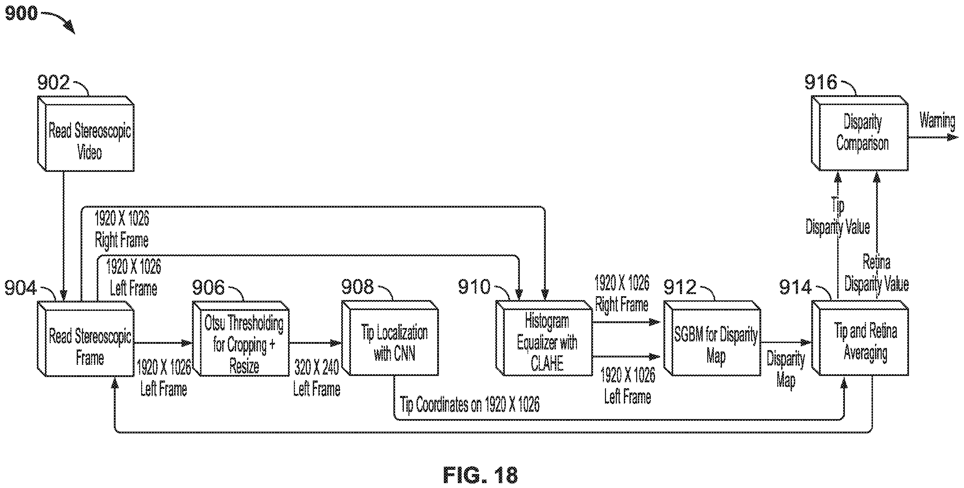

Fig. 18 depicts an example pipeline 900 for analyzing image data from an imaging system 802 comprising a digital stereo microscope (or other stereo image source). The pipeline 900 employs a unique combination of several computer vision techniques and a convolutional neural network (CNN) to provide additional depth information using as input a series of stereoscopic images (e.g., a stereoscopic video feed). In detail, the pipeline 900 receives stereoscopic video (block 902) for example, of an ophthalmic surgery, received as a stream of data comprising consecutive images from each of two (or more) cameras. It then reads a single stereoscopic frame from the stereoscopic video and pre-processes the stereoscopic frame to split left and right view and remove from each frame the logo of the framework employed by the surgical microscope to encode the stereoscopic video (block 904). At this point, Otsu Thresholding is employed to crop a copy of the left frame to 1368 × 1026 to center the region of interest (ROI) represented by the circular view of the fundus and remove the areas of black pixels near the edges that are useless, and the frame is resized to 320 × 240 (block 906). While Adaptive Thresholding is basic and simply implemented, it proved to be too unreliable in finding a proper threshold value. As a result, Otsu Thresholding is preferred because of its ability to easily and reliably divide peaks in bi-modal histograms.

Otsu Thresholding is a histogram-based method, meaning that it leverages the image histogram to choose a proper value for the threshold. Otsu Thresholding tries to minimize the intra-class intensity variance of pixels by looking at the probability of each intensity value in the histogram. The algorithm is the following: (1) compute the image histogram and the probability $P(i)$ of each intensity value $i$; (2) for each threshold value $t$ between 1 and 255, compute

$$\sigma^2(t) = q_1(t)\sigma_1^2(t) + q_2(t)\sigma_2^2(t)$$

where $\sigma^2(t)$ is the intra-class variance of two classes expressed as weighted sum of the variance of the two classes, $\sigma^2_1(t)$ is the variance of the first class, $\sigma^2_2(t)$ is the variance of the second class and

$$q_1(t) = \sum_{i=0}^{t} P(i)$$

$$q_2(t) = \sum_{i=t+1}^{255} P(i)$$

(3) the Otsu's threshold corresponds to the value $\bar{t}$ producing the highest intra-class variance $\sigma^2(t)$.

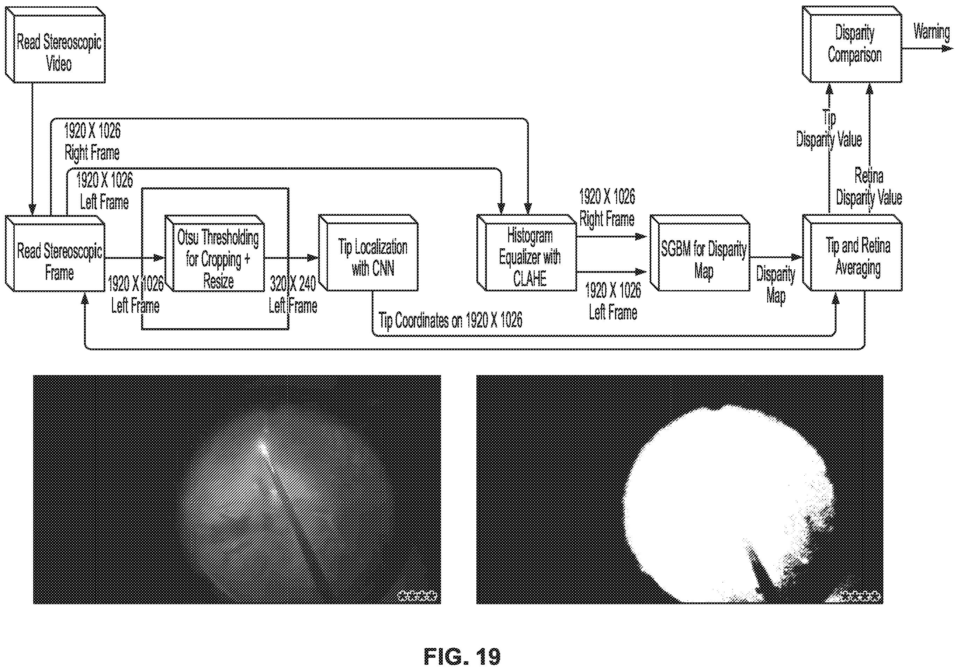

Otsu Thresholding works particularly well with bi-modal images, i.e. images whose histograms present two clearly-separated peaks. However, when the valley between the two peaks in the histogram is corrupted, due to noise or small foreground area with respect to the background, Otsu Thresholding presents some limitations in computing the right threshold value. Fig. 19 depicts two images that show an example of the action of Otsu threshholding. The left image shows the stereoscopic frame, while the right image shows the output of the Otsu threshholding step 906.

Once the left frame has been resized, it is fed into the Convolutional Neural Network to locate the tip of any surgical instrument tip in the surgical field, if any (block 908). The outcome of this step is a tuple containing the coordinates of the tip of the surgical instrument in the left frame. This step will be described in further detail below.

Thereafter, both the original left and right frames are pre-processed with Contrast Limited Adaptive Histogram Equalization (CLAHE) (block 910) just before computing the disparity map of the scene with Semi-Global Block Matching (SGBM) (block 912). CLAHE does not suffer from the noise-amplification issues of adaptive histogram equalization, but still applies local histogram equalization, which proved more effective for the present embodiments. SGBM, meanwhile, is a stereo matching algorithm that finds the correspondences between the left and right frames. SGBM is widely adopted for real-time applications thanks to its tradeoff between good quality and run-time. The peculiarity of SGBM is that it looks for corresponding points within a subset of the image. Given a pair of rectified images and point $p$ in the left image with coordinates $(x,y)$, SGBM looks for the match $p'$ in the right image as

$$\{x' \geq x \wedge \leq x + D\}$$

where D is the maximum allowed disparity. This limited search space reduces dramatically the run-time.

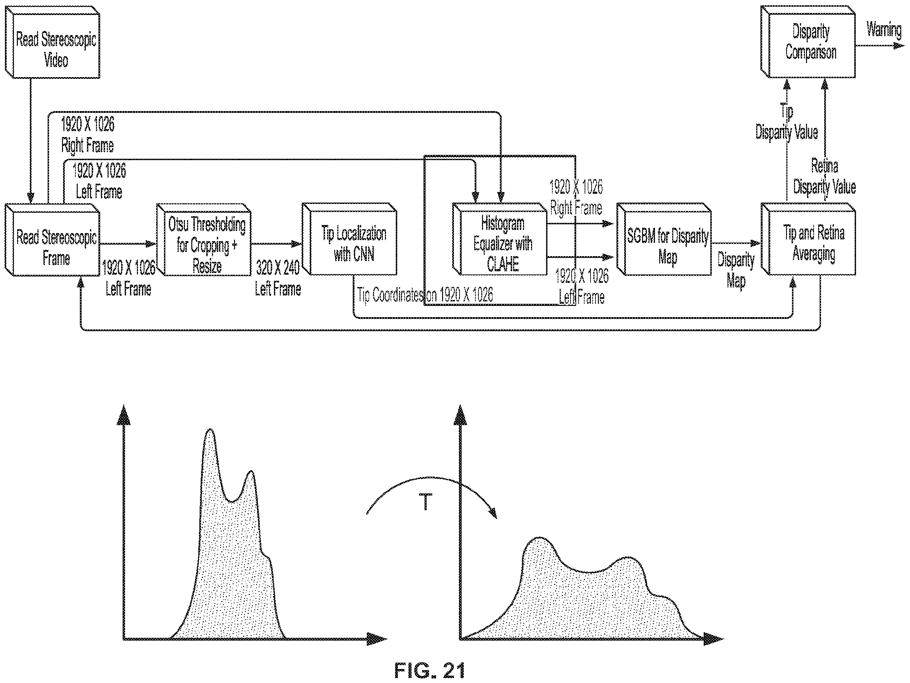

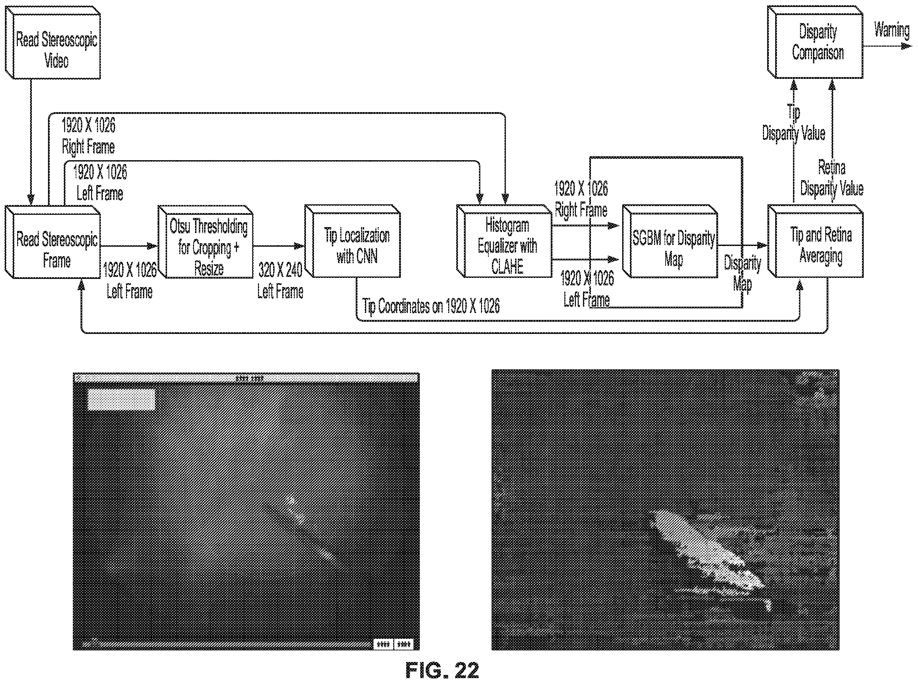

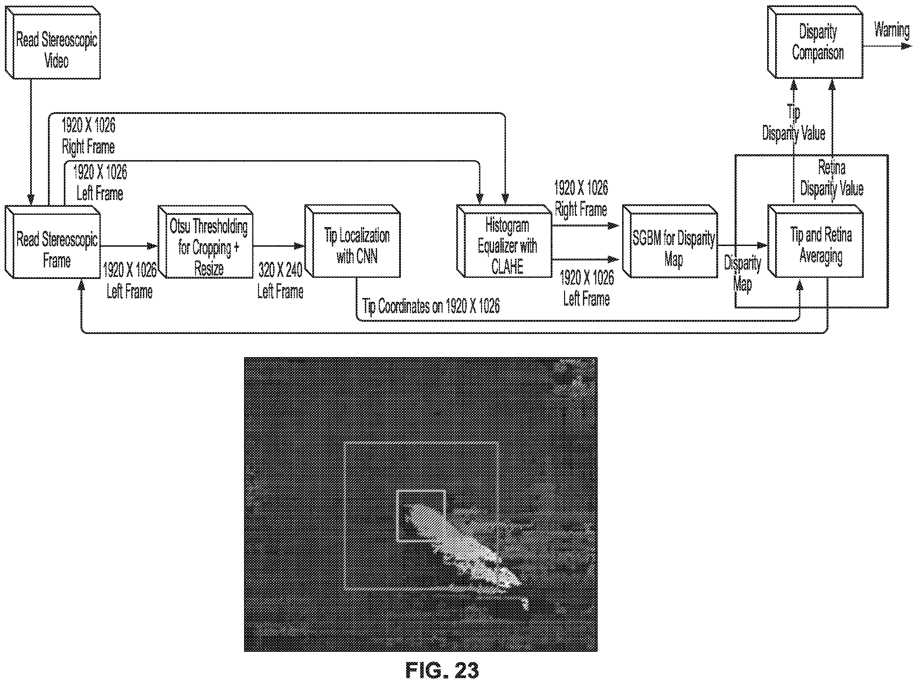

Fig. 21 depicts an example transformation accomplished during the histogram equalization using CLAHE (block 910). The input to the CLAHE processing step is depicted on the left, and the output is depicted on the right. Similarly, Fig. 22 depicts an example input and output of the SGBM algorithm (block 912). The input image is depicted on the left, and the output depicted on the right. Because relative disparities are independent from the camera's intrinsic parameters, transformation from relative disparity (in percentage) to absolute distance (in mm) between the instrument and the retina is not performed in order to avoid dependence on the microscope employed and relative values are used. In the case that stereomicroscope camera intrinsic parameters are known and available in real-time by a communication established between the microscope controller and the computer processing the stereo images, then absolute distance (in mm) between the instrument and the retina could be computed. The output of this step is a disparity map of the entire scene where landmarks are in the same coordinates system of the left frame, because the stereo matching algorithm uses the left frame as the reference. This allows applying the coordinates estimated in the previous step to look for the disparity values of the pixels belonging to the tip of the surgical instrument and the background retina. To do this an average disparity value of the tip of the surgical instrument is computed by averaging the disparity values around the estimated coordinates with a mask and Gaussian weights (block 914). A similar computation is performed to compute an average disparity value for the retina. The difference is the size of the mask used which is larger and the weights that should be such to exclude the pixels belonging to the instrument since it will also appear in this mask. Fig. 23 depicts an example output of the tip and retina averaging step (block 914).

Having a unique disparity value for both the tip of the surgical instrument and the retina, a comparison can be performed (block 916). Warnings may be issued if the comparison outputs a value below a fixed safety threshold. In embodiments, to provide a retina average disparity value more resilient with respect to noise and available even when the retina results occluded, the 120 most recent disparity values estimated for the retina are stored and their running mean computed. All values distant from this mean are discarded as outliers. This is possible since the retina surface does not move significantly during the entire video and, in particular, among frames.

On the other hand, this same approach cannot be implemented for the tip of the surgical instrument, as the latter moves much more during the surgeries.

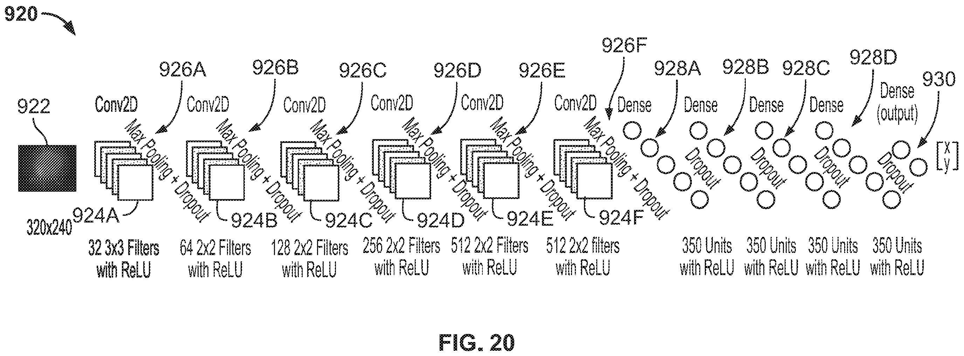

The structure 920 of an example CNN for localizing the surgical instrument tip (block 910) is depicted in Fig. 20. While standard computer vision techniques, such as the Hough transform, color thresholding, and edge detection are possible methods for tip localization (and therefore within the scope of the invention), these techniques all suffer from a lack of generalization and sensitivity of their respective results to variance in the data. For instance, the Hough transform may be effective if all of the instruments in the image data are identical (e.g., straight pieces of steel). Unfortunately, this is not the case as there exist non-linear (e.g., hooked) instruments. Color thresholding, meanwhile, could produce valuable results if surgical instruments were always the same color, but this is not the case and harsh lighting conditions in the surgical field produce red, yellow, and blue shades. Edge detection will suffice where the edges in the image frames are extremely sharp, but otherwise fall short of expectations. Accordingly, the preferred method to produce reliable identification of surgical instrument tips in the image data of surgical fields is a deep learning method.

The artificial neural network depicted in Fig. 20 is a CNN coupled with a Fully-Connected Feed-Forward Neural Network (FC-FFNN) to perform regression. The opening CNN extracts features from the image data of the surgical field. The FC-FFNN is then employed to leverage those extracted features and learn the coordinates of the surgical instrument's tip in the frame.

The input shape for the CNN is (320, 240, 3), corresponding to RGB image data 922. The convolutional section is built with six two-dimensional convolutional layers 924A-F, each followed by corresponding layers of Max Pooling and of Dropout 926A-F. The first convolutional layer 924A has 32 kernels with size (3, 3), ReLU activation function, and padding, so that no pixels are lost during convolution. Each of convolutional layers 924B-F has kernels of size (2, 2), and each doubles the respective number of kernels up to 512, with convolutional layers 924B-E having, respectively, 64, 128, 256, 512 kernels, and convolutional layer 924F having 512 kernels. Each of convolutional layers 924B-F maintains the ReLU activation function and padding. All of the max pooling and dropout layers 926A-F have a kernel size of (2, 2) and padding. The dropout probability is 0.05 for the first dropout layer 926A, 0.1 for the second dropout layer 926B, and 0.2 for each of dropout layers 926C-F. The Fully-Connected segment of the CNN consists of four dense hidden layers 928A-D, each having 350 neurons and ReLU activation function. The output layer 930 has two neurons with ReLU activation.

Of course, the CNN employed for tip localization (block 908) requires training. The input shape for the network is (320; 240; 3) representing RGB images with shape 320 240. RGB images are used instead of greyscale (1-channel) images to preserve the majority of the details in the images, because the colors contain useful information for the surgeon and for the CNN. A batch size (i.e., the number of samples considered at each step of the optimization) of 64 is employed. A variable learning rate having an initial value of 0.001, and is dynamically changed by the Adam optimizer, which is an implementation of a gradient descent algorithm that converges faster on large datasets.

Early stopping was deployed, with patience 15 epochs and model checkpoint with frequency 1, which stores the values of the weights each time a new minimum in the loss function is reached. This ensure to always have the best set of weights so far. The two output neurons 930 are configured with the ReLU activation function because we are performing a regression task on pixel coordinates and want neurons that can output non-negative real values, as pixels' coordinates range from 0 to *image width* and from 0 to *image height*. A custom loss function is employed to implement the Euclidean distance:

$$\sqrt{\sum_{i=0}^{n}(y_i - \hat{y}_i)^2}$$

where $y_i$ is the produced output, $\hat{y}_i$ is the expected value and $n$ is the number of samples. This minimizes the error distance between the predicted point and the ground truth.

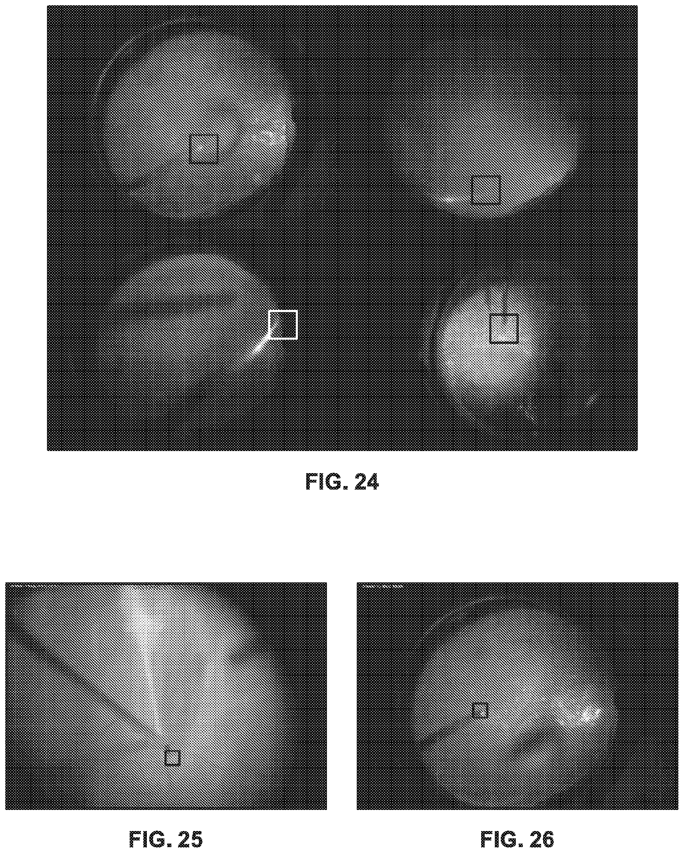

Figs. 24-26 show examples of the output of the example pipeline 900 acting on stereoscopic images received from the imaging system. In each, the pipeline 900 successfully identifies the tip of a surgical instrument in the image data of the surgical field, highlighting the tip of the surgical instrument in each frame with a box.

Feedback mechanisms

Various types of feedback may be provided to the surgeon in various embodiments of the IGSS 800. As described above, the IGSS 800 may include, as feedback devices 804, one or more of a display 810, a speaker 812, and a haptic feedback system 814. Though each the feedback devices 804 will be described individually, the feedback devices 804 may be included in various combinations in corresponding various embodiments. That is, in embodiments, the only implemented feedback device 804 is the display 810, while in other embodiments, the only implemented feedback device 804 is the speaker 812, and in still other embodiments, the only implemented feedback device 804 is the haptic feedback system 814. In any embodiment, a display 810, while not serving as a feedback device 804, may still be provided for allowing the surgeon to view the surgical field. In still other embodiments, the IGSS 800 may include the display 810 and the speaker 812, may include the display 810 and the haptic feedback system 814, may include the speaker 812 and the haptic feedback system 814, and/or may include all three of the display 810, the speaker 812, and the haptic feedback system 814.

As described above, the surgeon may receive feedback regarding aspects of the anatomy and/or the surgical procedure including, but not limited to: location of an identified tissue boundary; proximity of a surgical instrument tip to an identified tissue boundary; tissue deformation (e.g., resulting from manipulation of the tissue with a surgical instrument or movement of the surgical instrument within the surgical field); tissue volume; tissue attachments (e.g., retinal detachment); shear stress on the tissue; tissue movement; tissue position; exposed area of the tissue; and occluded area of the tissue, i.e., occlusion of any instrument with tissue.

Said feedback may be displayed, in embodiments, on the display 810, and may take various forms in various embodiments. The following non-limiting examples illustrate the manner in which feedback may be provided using the display 810:

An identified tissue boundary may be highlighted on the image data displayed on the display 810. An identified tissue cross-section may be highlighted on the image data displayed on the display 810.

An identified tissue boundary or cross-section may be highlighted in colors, brightness values, contrasts, or other displayed aspects, that change according to the state of a parameter (shear stress, deformation, volume, etc.) associated with the tissue, to indicate that the parameter is within desired limits, approaching a warning limit, or approaching a danger limit. The color, for example, may be green when the parameter is within a safe range, yellow when approaching a dangerous range, and red when extremely close to or in the dangerous range. The brightness and/or contrast could be adjusted in a similar manner. Additionally or alternatively, text warnings or status may be added to the images displayed on the display 810 to provide a state of the parameter.

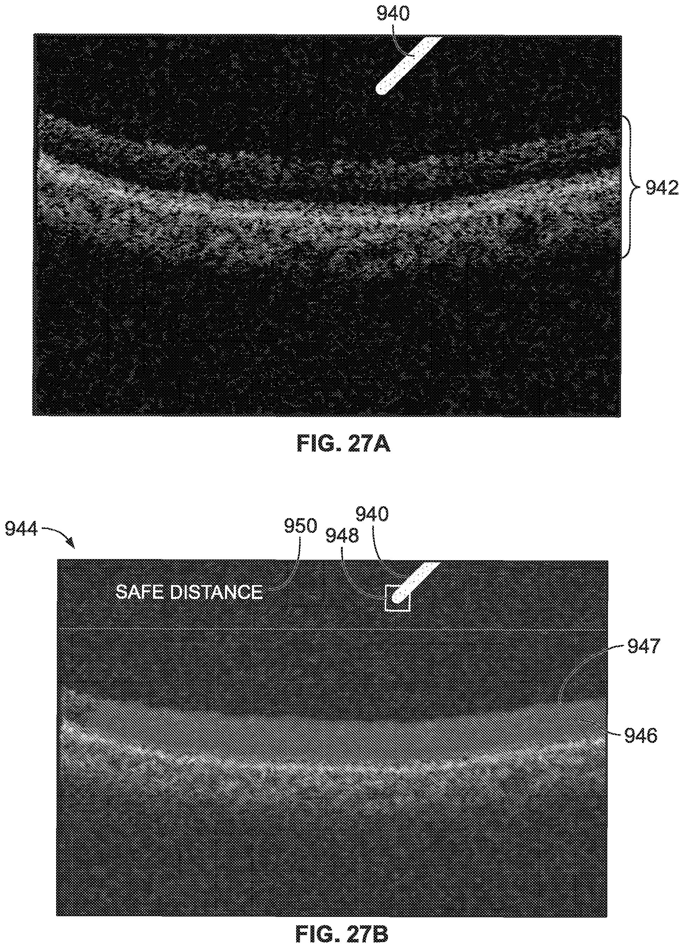

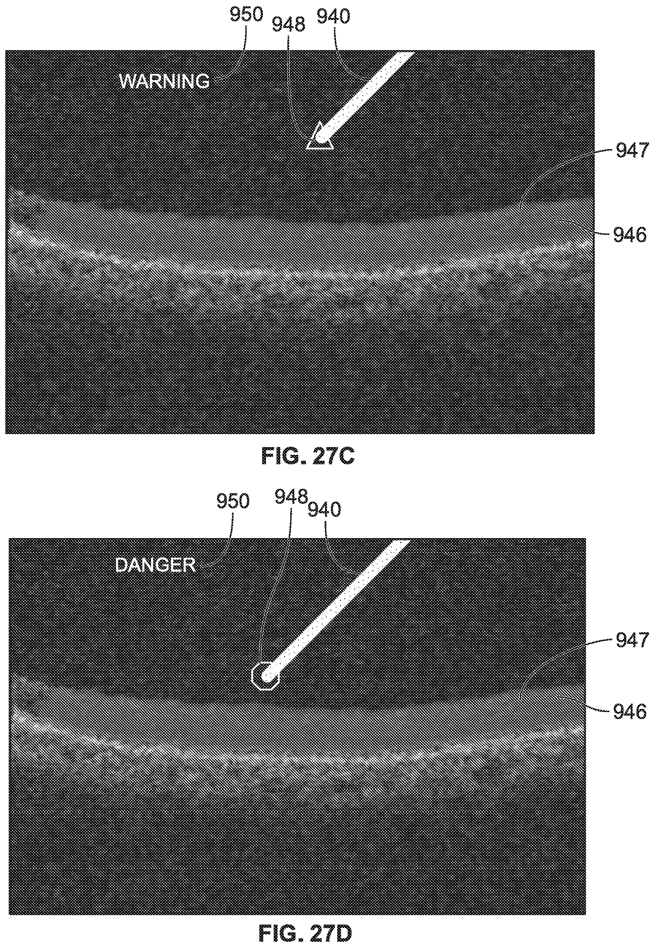

An identified tip of a surgical instrument may be highlighted in an image displayed by the display 810. For example, a box or other indicia may be provided around the tip of the surgical instrument, as depicted in Figs. 24-26. The box or indicia may vary in color (e.g., green, yellow, red), shape (e.g., circle, triangle, octagon), brightness, contrast, thickness, or other aspect in order to indicate whether the tip of the surgical instrument is in a safe range from a tissue boundary, is approaching a dangerous proximity to the tissue boundary, is dangerously close to or touching the tissue, etc. Figs. 27A-D depict an example series of images that may be displayed on the display 810 implemented as a feedback device 804. In Fig. 27A, a raw image that might be received from an iOCT imaging system and displayed on the display 810 is shown. The image in Fig. 27A depicts a surgical instrument 940 and various tissue layers 942 that are not identified and do not have any boundaries marked. The image in Fig. 27B shows an augmented image 944 providing as feedback both an indication 946 of a retinal layer having a boundary 947, and an indication 948 of a tip of the surgical instrument 940. The indication 948 may be displayed in a particular color (e.g., green) and/or style (e.g., square with 1 pt line, square with a first brightness) to indicate that a determined distance between the tip of the surgical instrument 940 and the tissue boundary is sufficiently large that there is no danger to the tissue. As the tip of the surgical instrument 940 gets closer to the boundary 947 of the tissue 946, the indication 948 of the tip of the surgical instrument 940 may change to indicate that the tip of the surgical instrument 940 is approaching an area considered to be dangerously proximal to the tissue boundary 947. The indication 948 may, for example, be displayed in a different color (e.g., yellow) and/or style (e.g., triangle with 1.5 pt line, triangle with a higher brightness), as depicted in Fig. 27C. As the tip of the surgical instrument 940 gets still closer to the boundary 947 of the tissue 946, the indication 948 of the tip of the surgical instrument 940 may change yet again to indicate that the tip of the surgical instrument 940 is approaching an area in which contact between the tip of the surgical instrument 940 and the tissue boundary 947 is imminent. The indication 948 may, for example, be displayed in yet a different color (e.g., red) and/or style (e.g., octagon with 2 pt line, octagon with a still higher brightness), as depicted in Fig. 27D. Various text may also (or alternatively) be displayed in varying size, emphasis, etc. For example, in Fig. 27B, a notation 950 indicates that the tip of the surgical instrument 940 is a "safe distance" from the tissue boundary 947. In Fig. 27C, the notation 950 is depicted in bold lettering as the message "WARNING." In Fig. 27D, the notation 950 is in bold and a larger font as the message "DANGER."

The feedback may also or alternatively be provided, in embodiments, via a speaker 812, and may take various forms in various embodiments. The following non-limiting examples illustrate the manner in which feedback may be provided using the speaker 812:

A parameter associated with an identified tissue boundary or cross-section may be conveyed to the surgeon via the speaker 812, and may change according to the state of a parameter (shear stress, deformation, volume, etc.) associated with the tissue, to indicate that the parameter is within desired limits, approaching a warning limit, or approaching a danger limit. The speaker 812, for example, annunciate verbally when the parameter is within a safe range, when approaching a dangerous range, and when extremely close to or in the dangerous range. Alternatively, the speaker 812 may provide tone that changes in pitch and/or volume and/or waveform (e.g., saw tooth, sine, square, etc.) to provide an indication to the surgeon of the state of the parameter.

A tone associated with the distance between an identified tip of a surgical instrument and a tissue boundary may be output by the speaker 812. The tone may vary in pitch (e.g., 500 Hz, 750 Hz, 1000 Hz), wave form (e.g., sine, square, saw tooth), and/or volume (e.g., 3 dB, 6dB, 9dB) in order to indicate whether the tip of the surgical instrument is in a safe range from a tissue boundary, is approaching a dangerous proximity to the tissue boundary, is dangerously close to or touching the tissue, etc.

The feedback may also or alternatively be provided, in embodiments, via a haptic feedback system 814, and may take various forms in various embodiments. The haptic feedback system 814 may be physically coupled to the surgical instrument, communicatively coupled to the surgical instrument, and/or non-coupled to the surgical instrument, but physically coupled to the surgeon. In embodiments, for example, the haptic feedback system 814 may comprise a joystick or other control device manipulated by the surgeon to cause movement of the surgical instrument (e.g., by a remote robotic platform). In other embodiments, the haptic feedback system 814 may be physically coupled to the surgical instrument and the surgeon may move the surgical instrument directly. For instance, the haptic feedback system 814 may comprise a grip or coupling device that follows the surgical instrument as it is manipulated by the surgeon, and provides feedback by vibrating, providing resistance against the forces applied by the surgeon, or the like. The following non-limiting examples illustrate the manner in which feedback may be provided using the haptic feedback system 814:

The haptic feedback system 814 may provide increased resistance to movement of the surgical instrument as a parameter (e.g., shear stress, deformation, volume, etc.) associated with an identified tissue boundary or cross-section changes, to indicate that the parameter is within approaching a warning limit or approaching a danger limit. For example, as a retinal tissue is pulled, the determined (e.g., estimated or calculated from changes of the area size of the segmented retina) shear stresses or deformation of the retina may increase to levels approaching a dangerous level, and the haptic feedback system 814 may provide proportionately (or disproportionately) more resistance as the parameter approaches the dangerous level. The haptic feedback system 814 may, in embodiments, prevent further movement or applied pressure to the surgical instrument when the parameter reaches the dangerous level, to prevent or make very difficult additional movement of the surgical instrument that could cause damage to the tissue.

The haptic feedback system 814 may provide increased resistance to movement of the surgical instrument as an identified tip of a surgical instrument approaches a tissue boundary (e.g., as a surgical instrument approaches a surface of the retina), to indicate that the tip of the surgical instrument is coming within a predefined distance from the retina surface. For example, as the tip of the surgical instrument comes within several millimeters of the retinal surface, the haptic feedback system 814 may provide proportionately (or disproportionately) more resistance. The haptic feedback system 814 may, in embodiments, prevent further movement or applied pressure to the surgical instrument when the tip of the surgical instrument is at a boundary of an "exclusion zone" (e.g., within 1 mm of the retinal surface), to prevent or make very difficult additional movement of the surgical instrument that could cause the tip of the surgical instrument to contact and/or damage the tissue.

Alternatively or additionally, the haptic feedback system 814 may vibrate in a manner such that the surgeon can perceive the vibration but the vibration does not affect the surgical instrument (e.g., by having a separate vibrating component worn by the surgeon, or by physically decoupling the surgical instrument from the haptic feedback system 814, as in the case of a robotic system). Such vibrations may be triggered by any parameter described herein approaching any desired limit.

In some embodiments, particularly those in which the haptic feedback system 814 remotely controls the surgical instrument, the haptic feedback system 814 may be configured to dampen the motion of the surgeon. In particular, as the tip of the surgical instrument approaches a tissue boundary, or as a parameter associated with the tissue or tissue boundary approaches a predefined limit, the haptic feedback system 814 may increase the amount of movement required as an input into the haptic feedback system 814 to move the surgical instrument a fixed distance or, conversely, may move the surgical instrument in smaller increments for a given input movement at the haptic feedback system 814. Alternatively, the haptic feedback system 814 may withdraw the surgical instrument from the proximal tissue, or withdraw the instrument from the surgical field (e.g., the eye), if collision with tissue is imminent, for example, in the case of inadvertent movement on the part of the surgical patient, or may turn off suction, for example, if the system determines that the stresses on, or deformation of, the tissue exceed a predetermined threshold. The relationship between the movement of the surgical instrument and the input to the haptic feedback system 814 may be adjusted proportionally inversely to the distance from a predetermined limit, exclusion zone, or tissue boundary, for example.

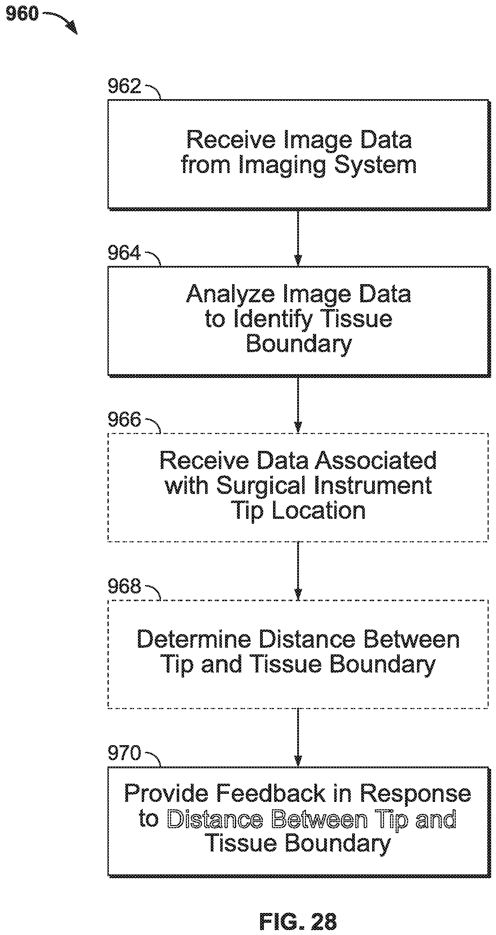

Fig. 28 is a flow chart depicting an example method 960 of implementing the IGSS 800. In the method 960, the processor 806 receives image data from the imaging system 802 (block 962). The image data are analyzed by the processor 806 to identify a tissue boundary (block 964) as described throughout this disclosure. The processor 806 provides feedback in response to the identified tissue boundary (block 970). In embodiments, the feedback provided may be feedback with respect to a parameter, such as tissue area, tissue volume, shear stress, etc., determined according to the identified tissue boundary. In other embodiments, the processor 806 receives data associated with surgical instrument tip location (block 966) and determines a distance between the tip of the surgical instrument and the identified tissue boundary (block 968) as described throughout this specification. In such embodiments, the feedback provided (block 970) may be in response to the distance between the tip and the tissue boundary.

The following list of aspects reflects a variety of the embodiments explicitly contemplated by the present application. Those of ordinary skill in the art will readily appreciate that the aspects below are neither limiting of the embodiments disclosed herein, nor exhaustive of all of the embodiments conceivable from the disclosure above, but are instead meant to be exemplary in nature.

1. A surgical guidance system comprising: a processor; a display communicatively coupled to the processor; an imaging system communicatively coupled to the processor; a memory device, communicatively coupled to the processor, and storing instructions, executable by the processor, to cause the processor to: receive, from the imaging system, real-time image data of a surgical field during a surgical procedure; analyze the image data in real-time to identify a tissue boundary present in the image data of the surgical field; and provide real-time visual, auditory, and/or haptic feedback in response to the identified tissue boundary.

1A. The surgical guidance system of aspect 1, wherein the instructions stored on the memory device are further executable to cause the processor to: receive data from which a position of a tip of a surgical instrument may be determined, the received data comprising either the real-time image data from the imaging system or sensor data from one or more sensors physically coupled to the surgical instrument; and determine, in real-time, a distance between a tip of the surgical instrument and the tissue boundary and wherein providing real-time visual, auditory, and/or haptic feedback in response to the identified tissue boundary comprises providing the real-time visual, auditory, and/or haptic feedback in response to the distance between the tip of the surgical instrument and the tissue boundary.

2. The surgical guidance system of either aspect 1 or aspect 1A, wherein: the surgical guidance system is a microsurgical guidance system; the surgical field is a microsurgical field; and the surgical instrument is a microsurgical instrument.

3. The surgical guidance system of any one of aspects 1 to 2, wherein the imaging system comprises a system performing intraoperative optical coherence tomography (iOCT).

4. The surgical guidance system of any one of aspects 1 to 3, wherein the imaging system comprises a digital stereoscopic vision system.

5. The surgical guidance system of any one of aspects 1 to 4, wherein receiving real-time image data of a surgical field during a surgical procedure comprises receiving real-time image data of intra-ocular anatomy during an ophthalmic surgery.

6. The surgical guidance system of any one of aspects 1 to 5, wherein analyzing the image data in real-time to identify a tissue boundary present in the image data of the surgical field comprises inputting the image data into a trained artificial intelligence (AI) model configured to identify tissue boundaries and/or tissue types based on the image data.

7. The surgical guidance system of aspect 6, wherein inputting the image data into a trained AI model configured to identify tissue boundaries and/or tissue types comprises inputting into the trained AI model configured to identify tissue boundaries and/or tissue types pre-processed image data.

8. The surgical guidance system of aspect 7, wherein inputting into the trained AI model configured to identify tissue boundaries and/or tissue types pre-processed image data comprises inputting into the trained AI model configured to identify tissue boundaries and/or tissue types image data on which one or more of the following processes has been implemented: compression, cropping, filtering, normalization, contrast adjustment, brightness adjustment, conversion to greyscale, downsampling, and/or upsampling.

9. The surgical guidance system of any one of aspects 1 to 8, wherein receiving data from which the position of the tip of the surgical instrument may be determined comprises receiving the real-time image data from the imaging system.

10. The surgical guidance system of aspect 9, wherein the instructions are further executable by the processor to analyze, in real-time, the image data received from the imaging system to identify in the surgical field the tip of the surgical instrument.

11. The surgical guidance system of aspect 10, wherein identifying in the surgical field the tip of the surgical instrument comprises inputting the image data into a trained artificial intelligence (AI) model configured to identify the tip of the surgical instrument based on the image data.

12. The surgical guidance system of aspect 11, wherein inputting the image data into a trained AI model configured to identify the tip of the surgical instrument comprises inputting into the trained AI model configured to identify the tip of the surgical instrument pre-processed image data.

13. The surgical guidance system of aspect 12, wherein inputting into the trained AI model configured to identify the tip of the surgical instrument pre-processed image data comprises inputting into the trained AI model configured to identify the tip of the surgical instrument image data on which one or more of the following processes has been implemented: compression, cropping, filtering, normalization, contrast adjustment, brightness adjustment, conversion to greyscale, downsampling, and/or upsampling.

14. The surgical guidance system of any one of aspects 1 to 13, wherein receiving data from which the position of the tip of the surgical instrument may be determined comprises receiving sensor data from one or more sensors physically coupled to the surgical instrument.

15. The surgical guidance system of any one of aspects 1 to 14, wherein the system provides real-time visual feedback in response to the distance between the tip of the surgical instrument and the tissue boundary, and wherein providing real-time visual feedback comprises one or more of: identifying on a displayed image of the surgical field the tip of the surgical instrument; changing a color associated with the tip of the surgical instrument according to the distance between the tip of the surgical instrument and the tissue boundary; changing a brightness associated with the tip of the surgical instrument according to the distance between the tip of the surgical instrument and the tissue boundary; placing text on a displayed image of the surgical field to warn of proximity of the surgical instrument to the tissue boundary; or augmenting a displayed image of the surgical field to highlight the tissue or the tissue boundary.

16. The surgical guidance system of any one of aspects 1 to 15, wherein the system provides real-time auditory feedback in response to the distance between the tip of the surgical instrument and the tissue boundary, and wherein providing real-time auditory feedback comprises one or more of: annunciating a verbal warning that the tip of the surgical instrument is approaching the tissue boundary; providing an audible tone or alarm in response to the proximity of the tip of the surgical instrument to the tissue boundary; changing a pitch of an audible tone or alarm in response to the proximity of the tip of the surgical instrument to the tissue boundary; or changing a volume of an audible tone or alarm in response to the proximity of the tip of the surgical instrument to the tissue boundary.

17. The surgical guidance system of any one of aspects 1 to 16, wherein the system provides real-time haptic feedback in response to the distance between the tip of the surgical instrument and the issue boundary, wherein the system further comprises a haptic device, and wherein providing real-time haptic feedback comprises one or more of: vibrating the haptic device as the tip of the surgical instrument crosses a threshold distance from the tissue boundary; causing the haptic device to increase resistance to movement of the tip of the surgical instrument in the direction of the tissue boundary as the tip of the surgical instrument approaches the tissue boundary; or causing, in a haptic device that is robotically controlling the movement of the surgical instrument, the haptic device to change the ratio of movement of the haptic device to movement of the tip of the surgical instrument as the tip of the surgical instrument approaches the tissue boundary.

18. The surgical guidance system of any one of aspects 1 to 17, further comprising displaying a parameter and/or a warning related to the deformation or compression of tissue in the surgical field, the parameter or warning determined according to the real-time image data.

19. The surgical guidance system of aspect 18, wherein the parameter and/or warning are associated with one or more of: a change in volume of an entity in the surgical field; and a calculated shear stress applied to a tissue in the surgical field.

20. The surgical guidance system of any one of aspects 1 to 19, wherein the imaging system includes both the system performing OCT and the digital stereoscopic vision system, and wherein the guidance system is operable to switch between the system performing OCT and the digital stereoscopic vision system during a single surgical procedure, as needed.

A. A memory device, communicatively coupled to a processor, and storing instructions, executable by the processor, to cause the processor to: receive, from an imaging device communicatively coupled to the processor, real-time image data of a surgical field during a surgical procedure; analyze the image data in real-time to identify a tissue boundary present in the image data of the surgical field; and provide real-time visual, auditory, and/or haptic feedback in response to the identified tissue boundary.

A+1A. The memory device of aspect A, wherein the instructions are further operable to cause the processor to: receive data from which a position of a tip of a surgical instrument may be determined, the received data comprising either the real-time image data from the imaging system or sensor data from one or more sensors physically coupled to the surgical instrument; and determine, in real-time, a distance between a tip of the surgical instrument and the tissue boundary; wherein providing real-time visual, auditory, and/or haptic feedback in response to the identified tissue boundary comprises providing the real-time visual, auditory, and/or haptic feedback in response to the distance between the tip of the surgical instrument and the tissue boundary.

A+1. The memory device of aspect A or aspect A+1A, wherein: the surgical guidance system is a microsurgical guidance system; the surgical field is a microsurgical field; and the surgical instrument is a microsurgical instrument.

A+2. The memory device of any one of aspects A to A+1, wherein receiving, from the imaging device communicatively coupled to the processor, real-time image data comprises receiving the real-time image data from a system performing optical coherence tomography (OCT).

A+3. The memory device of any one of aspects A to A+2, wherein receiving, from the imaging device communicatively coupled to the processor, real-time image data comprises receiving the real-time image data from a digital stereoscopic vision system.

A+4. The memory device of any one of aspects A to A+3, wherein receiving real-time image data of a surgical field during a surgical procedure comprises receiving real-time image data of intra-ocular anatomy during an ophthalmic surgery.

A+5. The memory device of any one of aspects A to A+4, wherein analyzing the image data in real-time to identify a tissue boundary present in the image data of the surgical field comprises inputting the image data into a trained artificial intelligence (AI) model configured to identify tissue boundaries and/or tissue types based on the image data.

A+6. The memory device of aspect A+5, wherein inputting the image data into a trained AI model configured to identify tissue boundaries and/or tissue types comprises inputting into the trained AI model configured to identify tissue boundaries and/or tissue types pre-processed image data.

A+7. The memory device of aspect A+6, wherein inputting into the trained AI model configured to identify tissue boundaries and/or tissue types pre-processed image data comprises inputting into the trained AI model configured to identify tissue boundaries and/or tissue types image data on which one or more of the following processes has been implemented: compression, cropping, filtering, normalization, contrast adjustment, brightness adjustment, conversion to greyscale, downsampling, and/or upsampling.

A+8. The memory device of any one of aspects A to A+7, wherein receiving data from which the position of the tip of the surgical instrument may be determined comprises receiving the real-time image data from the imaging system.

A+9. The memory device of aspect A+8, further comprising analyzing, in real-time, the image data received from the imaging system to identify in the surgical field the tip of the surgical instrument.

A+10. The memory device of aspect A+9, wherein identifying in the surgical field the tip of the surgical instrument comprises inputting the image data into a trained artificial intelligence (AI) model configured to identify the tip of the surgical instrument based on the image data.

A+11. The memory device of aspect A+10, wherein inputting the image data into a trained AI model configured to identify the tip of the surgical instrument comprises inputting into the trained AI model configured to identify the tip of the surgical instrument pre-processed image data.

A+12. The memory device of aspect A+11, wherein inputting into the trained AI model configured to identify the tip of the surgical instrument pre-processed image data comprises inputting into the trained AI model configured to identify the tip of the surgical instrument image data on which one or more of the following processes has been implemented: compression, cropping, filtering, normalization, contrast adjustment, brightness adjustment, conversion to greyscale, downsampling, and/or upsampling.

A+13. The memory device of any one of aspects A to a+12, wherein receiving data from which the position of the tip of the surgical instrument may be determined comprises receiving sensor data from one or more sensors physically coupled to the surgical instrument.

A+14. The memory device of any one of aspects A to A+13, wherein the system provides real-time visual feedback in response to the distance between the tip of the surgical instrument and the tissue boundary, and wherein providing real-time visual feedback comprises one or more of: identifying on a displayed image of the surgical field the tip of the surgical instrument; changing a color associated with the tip of the surgical instrument according to the distance between the tip of the surgical instrument and the tissue boundary; changing a brightness associated with the tip of the surgical instrument according to the distance between the tip of the surgical instrument and the tissue boundary; placing text on a displayed image of the surgical field to warn of proximity of the surgical instrument to the tissue boundary; or augmenting a displayed image of the surgical field to highlight the tissue or the tissue boundary.

A+15. The memory device of any one of aspects A to A+14, wherein the system provides real-time auditory feedback in response to the distance between the tip of the surgical instrument and the issue boundary, and wherein providing real-time auditory feedback comprises one or more of: annunciating a verbal warning that the tip of the surgical instrument is approaching the tissue boundary; providing an audible tone or alarm in response to the proximity of the tip of the surgical instrument to the tissue boundary; changing a pitch of an audible tone or alarm in response to the proximity of the tip of the surgical instrument to the tissue boundary; or changing a volume of an audible tone or alarm in response to the proximity of the tip of the surgical instrument to the tissue boundary.

A+16. The memory device of any one of aspects A to A+15, wherein the system provides real-time haptic feedback in response to the distance between the tip of the surgical instrument and the issue boundary, wherein the system further comprises a haptic device, and wherein providing real-time haptic feedback comprises one or more of: vibrating the haptic device as the tip of the surgical instrument crosses a threshold distance from the tissue boundary; causing the haptic device to increase resistance to movement of the tip of the surgical instrument in the direction of the tissue boundary as the tip of the surgical instrument approaches the tissue boundary; or causing, in a haptic device that is robotically controlling the movement of the surgical device, the haptic device to change the ratio of movement of the haptic device to movement of the tip of the surgical instrument as the tip of the surgical instrument approaches the tissue boundary.

A+17. The memory device of any one of aspects A to A+16, further comprising displaying a parameter and/or a warning related to the deformation or compression of tissue in the surgical field, the parameter or warning determined according to the real-time image data.

A+18. The memory device of aspect A+17, wherein the parameter and/or warning are associated with one or more of: a change in volume of an entity in the surgical field; and a calculated shear stress applied to a tissue in the surgical field.

A+19. The memory device of any one of aspects A to A+18, wherein the imaging system includes both the system performing OCT and the digital stereoscopic vision system, and wherein the guidance system is operable to switch between the system performing OCT and the digital stereoscopic vision system during a single surgical procedure, as needed.

B. An artificial intelligence (AI)-based surgical guidance system configured to provide real-time feedback to a surgeon during a surgical procedure, the system comprising: a first trained AI model configured to receive real-time images of a surgical field and to identify, in real-time, within the images, one or more tissue boundaries; an analysis module configured to: receive, from the first trained AI model, data indicating the one or more tissue boundaries identified by the first trained AI model; receive data indicating the position of the tip of the surgical instrument; determine, in real-time, from the data received from the first trained AI model and the data indicating the position of the tip of the surgical instrument, a distance between the tip of the surgical instrument and one of the one or more tissue boundaries; provide real-time visual, auditory, and/or haptic feedback to the surgeon based on the determined distance between the tip of the surgical instrument and the one of the one or more tissue boundaries.

B+1. The AI-based surgical guidance system of aspect B, further comprising a second trained AI model configured to receive the real-time images of the surgical field, and to identify, in real-time, a tip of a surgical instrument in the surgical field, and wherein receiving data indicating the position of the tip of the surgical instrument comprises receiving the data indicating the position of the tip of the surgical instrument from the second trained AI model.

B+2. The AI-based surgical guidance system of aspect B, further comprising one or more sensors providing data associated with the position and/or movement of the surgical instrument, and wherein receiving data indicating the position of the tip of the surgical instrument comprises receiving data from the one or more sensors.

B+3. The AI-based surgical guidance system of any one of aspects B to B+2, wherein: the surgical guidance system is a microsurgical guidance system; the surgical field is a microsurgical field; and the surgical instrument is a microsurgical instrument.

B+4. The AI-based surgical guidance system of any one of aspects B to B+3, wherein the real-time images are received from an optical coherence tomography (OCT) system.

B+5. The AI-based surgical guidance system of any one of aspects B to B+4, wherein the real-time images are received from a digital stereoscopic vision system.

B+6. The AI-based surgical guidance system of any one of aspects B to B+5, wherein receiving real-time image data of a surgical field during a surgical procedure comprises receiving real-time image data of intra-ocular anatomy during an ophthalmic surgery.

B+7. The AI-based surgical guidance system of any one of aspects B to B+6, wherein the system provides real-time visual feedback in response to the distance between the tip of the surgical instrument and the tissue boundary, and wherein providing real-time visual feedback comprises one or more of: identifying on a displayed image of the surgical field the tip of the surgical instrument; changing a color associated with the tip of the surgical instrument according to the distance between the tip of the surgical instrument and the tissue boundary; changing a brightness associated with the tip of the surgical instrument according to the distance between the tip of the surgical instrument and the tissue boundary; placing text on a displayed image of the surgical field to warn of proximity of the surgical instrument to the tissue boundary; or augmenting a displayed image of the surgical field to highlight the tissue or the tissue boundary.

B+8. The AI-based surgical guidance system of any one of aspects B to B+7, wherein the system provides real-time auditory feedback in response to the distance between the tip of the surgical instrument and the issue boundary, and wherein providing real-time auditory feedback comprises one or more of: annunciating a verbal warning that the tip of the surgical instrument is approaching the tissue boundary; providing an audible tone or alarm in response to the proximity of the tip of the surgical instrument to the tissue boundary; changing a tone of an audible tone or alarm in response to the proximity of the tip of the surgical instrument to the tissue boundary; or changing a volume of an audible tone or alarm in response to the proximity of the tip of the surgical instrument to the tissue boundary.

B+9. The AI-based surgical guidance system of any one of aspects B to B+8, wherein the system provides real-time haptic feedback in response to the distance between the tip of the surgical instrument and the issue boundary, wherein the system further comprises a haptic device, and wherein providing real-time haptic feedback comprises one or more of: vibrating the haptic device as the tip of the surgical instrument crosses a threshold distance from the tissue boundary; causing the haptic device to increase resistance to movement of the tip of the surgical instrument in the direction of the tissue boundary as the tip of the surgical instrument approaches the tissue boundary; or causing, in a haptic device that is robotically controlling the movement of the surgical device, the haptic device to change the ratio of movement of the haptic device to movement of the tip of the surgical instrument as the tip of the surgical instrument approaches the tissue boundary.

B+10. The AI-based surgical guidance system of any one of aspects B to B+9, wherein the analysis module is further configured to cause a display to show a parameter and/or a warning related to the deformation or compression of tissue in the surgical field, the parameter or warning determined according to the real-time image data.

B+11. The AI-based surgical guidance system of aspect B+10, wherein the parameter and/or warning are associated with one or more of: a change in volume of an entity in the surgical field; and a calculated shear stress applied to a tissue in the surgical field.

C. A method of providing real-time feedback to a surgeon during a microsurgical procedure, the method comprising: receiving real-time image data from an imaging system communicatively coupled to a processor; performing, in the processor, in real-time, segmentation of the image data to determine one or more tissue boundaries; performing, in the processor, in real-time, identification of a position of a tip of a microsurgical instrument relative to one of the one or more tissue boundaries; providing real-time visual, auditory, and/or haptic feedback to the surgeon based on the predetermined distance between the tip of the microsurgical instrument and the one of the one or more tissue boundaries.

D. A microsurgical guidance system comprising: a haptic device configured to provide direct feedback during a microsurgical procedure from different sources to a microsurgical instrument in real-time.

D+1. The microsurgical guidance system of aspect D, the system configured to receive real-time imaging data from an imaging and/or video capture device, the system configured to analyze and deliver direct modulation feedback concerning a position and/or function of the microsurgical instrumentation.

D+2. The microsurgical guidance system of aspect D+1, the system further comprising the OCT device.

D+3. The microsurgical guidance system of aspect D+1, further comprising a display operatively connected to the haptic device and the microsurgical instrument.

D+4. The microsurgical guidance system of aspect D+3, further comprising a feedback loop, whereby the location of the microsurgical instrument in relation to delicate ocular tissues is determined and the effect of instrument-tissue interactions is used in the analysis to guide surgical maneuvers with direct feedback.

D+5. The microsurgical guidance system of aspect D+4, wherein the feedback loop is operably connected to the imaging and/or video capture device, the microsurgical instrument, and the haptic device.

D+6. The microsurgical guidance system of aspect D+5, wherein the feedback loop generates an output including a tactile feedback signal, the output delivered to the haptic device, the output including graded resistance to movement of the microsurgical instrument approaching a threshold and/or exclusion zone defined as proximity to tissues or structures to be avoided.

D+7. The microsurgical guidance system of aspect D+5, wherein the feedback loop generates an output including a visual feedback signal, the visual feedback signal including changes to the appearance of the instruments and tissues on the display.

D+8. The microsurgical guidance system of aspect D+5, wherein the feedback loop generates an output including audible feedback to confer information to the surgeon regarding the position of surgical instruments or effect on ocular tissues and structures.

D+9. The system of aspect D, wherein precision microsurgical guidance is delivered via the haptic device by modulating the ratio of force applied to the microsurgical instrument during use in order to achieve a corresponding movement, which can include tremor dampening and fine control of the microsurgical instrument via the feedback.

D+10. The system of aspect D+3, wherein the direct feedback is generated based on segmented imaging data defining tissue boundaries and instrument position in real-time, the system analyzing, via a feedback loop operably connectable to a processor, the segmented imaging data, and generating a feedback output to the system including at least one of tactile, audio, and visual feedback.

D+11. The system of aspect D+5, further comprising a surgical instrument controller operably connected to the feedback loop, the system configured to either generate a warning alert and/or modulate function such as a change in grasping and/or vacuum and/or cutting and/or aspiration and/or application of electromagnetic (laser) or radiofrequency (diathermy) settings of the surgical instrument directly.

D+12. The system of aspect D+4, the display configured to receive real-time outputs from the feedback loop, the real-time outputs generated by the feedback loop based on instrument position data received from the haptic device, imaging data containing tissue/nerve information received from the OCT device, wherein the feedback loop analyzes the instrument position data, and the imaging data and produces a three dimensional output and/or an additional haptic feedback output in real-time.

E. A method of guiding a microsurgical instrument comprising: receiving image data in real-time from an imaging system operably connected, directly or indirectly, to the microsurgical instrument; segmenting and selecting slices or subsections from the image data and correlating the resulting image data with instrument position data via a processor operably connectable, directly or indirectly, to the microsurgical instrument to produce an output image, where the instrument position data can be acquired, at least in part, from a direct haptic feedback device; generating feedback in real-time via a feedback loop operably connected to the processor; sending the feedback to the microsurgical instrument via the direct haptic feedback device; and controlling the power of the microsurgical instrument, if needed.

E+1. The system of aspect E, further comprising an analysis loop configured to distinguish ocular tissue data from OCT data, and further configured to train the feedback loop via machine learning to discriminate among anatomical layers.

E+2. The system of aspect E, wherein image processing steps analyze and identify anatomical differences and changes in the segmented imaging data.

F. A microsurgical guidance system comprising: an imaging device configured to acquire tissue surround data in real-time; a haptic feedback device configured to acquire and send instrument position data derived from a surgical instrument; and a feedback loop configured to correlate tissue surround data and instrument position data, the feedback loop configured to analyze the tissue surround data and instrument position data to form an analyzed data output, the feedback loop configured to generate at least one of a display output, an audible output, and a haptic feedback output based on the analyzed data output.

F+1. The system of aspect F, the imaging device further comprising an image data acquisition device, the image data acquisition device producing real-time anatomical images, the feedback loop configured to merge the real-time anatomical images into a stereoscopic display containing depth information.

F+2. The system of aspect F, the feedback loop configured to identify tissue planes and/or anatomical structures of the real-time anatomical images from the imaging device output.

F+3. The system of aspect F, the feedback loop configured to generate an exclusion zone from the tissue structure and/or anatomic information and incorporate the exclusion zone into the output.

F+4. The system of aspect F+3, the visual output and/or the additional haptic feedback output in real-time configured to guide users of the system to avoid contacting and/or perturbing defined anatomical structures during a surgical procedure, or to inform tremor dampening, or changing ratio of applied force-to-movement of the surgical instrument mediated by the haptic feedback device, in some cases based on feedback force associated with the surgical instrument, the system configured to provide feedback for guiding fine movements that would be more difficult without the feedback, including control based on the power settings of the surgical instrument, and fine control of the surgical instrument in preventing removal of tissue, such that the system can be selectively configured to control both the location and the function of the surgical instrument in three dimensions, and to facilitate assisted surgical procedures and/or stabilize fine movements of the surgical instrument based on the feedback.

F+5. The system of aspect F+4, wherein the feedback loop is configured to generate the output within a latency period that allows for modulation of the surgical instruments in real-time.

F+6. The system of aspect F+2, the feedback loop configured to select two orthogonal images, if needed, to preserve the improved computational capabilities of the system, which depend on a location of the surgical instrument, the feedback loop further configured to process the selected images, and analyze the pixels that correspond to the intersection of both orthogonal images from the surgical instrument tip location received from the haptic feedback device all the way down across the selected anatomical images to the retina in the exclusion zone, considering neighboring pixels.

F+7. The system of aspect F+6, the three dimensional output formed based on the imaging data received from the imaging device, wherein the imaging device is a single OCT imaging device.

F+8. The system of aspect F+3, the additional retina layer information including a loop for informing the feedback loop with distinction parameters between clinically relevant tissue boundaries among anatomical structures, wherein the loop can be informed regarding the distinction parameters from a learned or stored information database, and/or form additional imaging information obtained from imaging data updates received at an earlier time.

G. In systems configured to guide microsurgical procedures, a method of expediting feedback to the surgeon in real-time, the method comprising: reducing a volume of an imaged area, if needed; performing image processing on a selected portion of the imaged area; targeting imaging processing of tissue boundaries while excluding internal structure during the imaging processing; and analyzing the targeted portion in view of anatomical changes to the images area and microsurgical instrument during the procedure.

H. A system for guiding microsurgical procedures, the system comprising: a microprocessor; an imaging system coupled to the microprocessor; a memory device coupled to the microprocessor, the memory device storing machine readable instructions that, when executed by the processor, cause the processor to: receive from the imaging system image data of an imaged area; reduce the volume of the imaged area, if needed; perform image processing on a selected portion of the imaged area target image processing of tissue boundaries while excluding internal structure during the image processing; and analyze the targeted portion in view of anatomical changes to the image area and microsurgical instrument during the procedure.

## Claims

1. An artificial intelligence (AI)-based surgical guidance system configured to provide real-time feedback to a surgeon during a surgical procedure, the system comprising:

   a first trained AI model configured to receive real-time images of a surgical field and to identify, in real-time, within the images, one or more tissue boundaries;
   an analysis module configured to:

   receive, from the first trained AI model, data indicating the one or more tissue boundaries identified by the first trained AI model;
   receive data indicating the position of the tip of the surgical instrument;
   determine, in real-time, from the data received from the first trained AI model and the data indicating the position of the tip of the surgical instrument, a distance between the tip of the surgical instrument and one of the one or more tissue boundaries; provide real-time visual, auditory, and/or haptic feedback to the surgeon based on the determined distance between the tip of the surgical instrument and the one of the one or more tissue boundaries.

2. The AI-based surgical guidance system of claim 1, further comprising a second trained AI model configured to receive the real-time images of the surgical field, and to identify, in real-time, a tip of a surgical instrument in the surgical field, and wherein receiving data indicating the position of the tip of the surgical instrument comprises receiving the data indicating the position of the tip of the surgical instrument from the second trained AI model.

3. The AI-based surgical guidance system of claim 1, further comprising one or more sensors providing data associated with the position and/or movement of the surgical instrument, and wherein receiving data indicating the position of the tip of the surgical instrument comprises receiving data from the one or more sensors

4. The AI-based surgical guidance system of any one of claims 1 to 3, wherein:

> the surgical guidance system is a microsurgical guidance system;
> the surgical field is a microsurgical field; and
> the surgical instrument is a microsurgical instrument.

5. The AI-based surgical guidance system of any one of claims 1 to 4, wherein the real-time images are received from an optical coherence tomography (OCT) system.

6. The AI-based surgical guidance system of any one of claims 1 to 5, wherein the real-time images are received from a digital stereoscopic vision system.

7. The AI-based surgical guidance system of any one of claims 1 to 6, wherein receiving real-time image data of a surgical field during a surgical procedure comprises receiving real-time image data of intra-ocular anatomy during an ophthalmic surgery.

8. The AI-based surgical guidance system of any one of claims 1 to 7,

> wherein the system provides real-time visual feedback in response to the distance between the tip of the surgical instrument and the tissue boundary, and wherein providing real-time visual feedback comprises one or more of:

>> identifying on a displayed image of the surgical field the tip of the surgical instrument; changing a color associated with the tip of the surgical instrument according to the distance between the tip of the surgical instrument and the tissue boundary; changing a brightness associated with the tip of the surgical instrument according to the distance between the tip of the surgical instrument and the tissue boundary; placing text on a displayed image of the surgical field to warn of proximity of the surgical instrument to the tissue boundary; or augmenting a displayed image of the surgical field to highlight the tissue or the tissue boundary.

9. The AI-based surgical guidance system of any one of claims 1 to 8,

> wherein the system provides real-time auditory feedback in response to the distance between the tip of the surgical instrument and the issue boundary, and wherein providing real-time auditory feedback comprises one or more of:

>> annunciating a verbal warning that the tip of the surgical instrument is approaching the tissue boundary; providing an audible tone or alarm in response to the proximity of the tip of the surgical instrument to the tissue boundary; changing a tone of an audible tone or alarm in response to the proximity of the tip of the surgical instrument to the tissue boundary; or changing a volume of an audible tone or alarm in response to the proximity of the tip of the surgical instrument to the tissue boundary.

10. The AI-based surgical guidance system of any one of claims 1 to 9,

> wherein the system provides real-time haptic feedback in response to the distance between the tip of the surgical instrument and the issue boundary, wherein the system further comprises a haptic device, and wherein providing real-time haptic feedback comprises one or more of:

vibrating the haptic device as the tip of the surgical instrument crosses a threshold distance from the tissue boundary;
causing the haptic device to increase resistance to movement of the tip of the surgical instrument in the direction of the tissue boundary as the tip of the surgical instrument approaches the tissue boundary; or
causing, in a haptic device that is robotically controlling the movement of the surgical device, the haptic device to change the ratio of movement of the haptic device to movement of the tip of the surgical instrument as the tip of the surgical instrument approaches the tissue boundary.

11. The AI-based surgical guidance system of any one of claims 1 to 10,
wherein the analysis module is further configured to cause a display to show a parameter and/or a warning related to the deformation or compression of tissue in the surgical field, the parameter or warning determined according to the real-time image data.

12. The AI-based surgical guidance system of claim 11, wherein the parameter and/or warning are associated with one or more of:

a change in volume of an entity in the surgical field; and
a calculated shear stress applied to a tissue in the surgical field.

100

**Microsurgical Guidance System**

110

Haptic
Feedback
Device

130

Display

120

Surgical
Instrument

**FIG. 1**

200

**Microsurgical Guidance System**

210

Haptic
Feedback
Device

250

Processor

240

Feedback
Loop

260

Imaging
Data
Acquisition
Device

**FIG. 2**

300

**Microsurgical Guidance System**

360

Image Data
Acquisition
Device

310

Haptic
Feedback
Device

340

Feedback
Loop

330

Display

350

Processor

320

Surgical
Instrument

**FIG. 3**

FIG. 4

EP 4 382 021 A2

FIG. 5

FIG. 6

FIG. 7

EP 4 382 021 A2

FIG. 8

**FIG. 9**

EP 4 382 021 A2

FIG. 10A

FIG. 10B

FIG. 11

FIG. 12

Input Image

Output Segmentation

Pooling

UpPooling

Convolution

Convolution 1x1 Filter

Copy and Crop

EP 4 382 021 A2

FIG. 13

FIG. 14B

FIG. 14D

FIG. 14A

FIG. 14C

FIG. 15A

FIG. 15B

FIG. 15C

FIG. 15D

FIG. 16A

FIG. 16B

FIG. 16C

FIG. 16D

**FIG. 17**

**FIG. 18**

**FIG. 19**

FIG. 20

FIG. 21

**FIG. 22**

EP 4 382 021 A2

**FIG. 23**

FIG. 24

FIG. 25

FIG. 26

940

942

**FIG. 27A**

944

950 948 940

SAFE DISTANCE

947

946

**FIG. 27B**

**FIG. 27C**

**FIG. 27D**

960

962

Receive Image Data
from Imaging System

964

Analyze Image Data
to Identify Tissue
Boundary

966

Receive Data Associated
with Surgical Instrument
Tip Location

968

Determine Distance Between
Tip and Tissue Boundary

970

Provide Feedback in Response
to Distance Between Tip and
Tissue Boundary

FIG. 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **G. ALDEGHI.** Thesis: Retinal Segmentation of Intraoperative B-Scan Optical Coherence Tomography Using Deep Learning. University of Illinois at Chicago
- **M. DE SILVESTRI.** Thesis: Real-time Haptic Guidance System for Retinal Surgery based on Intraoperative Optical Coherence Tomography. University of Illinois at Chicago
- **M. DIFATTA.** Thesis: Surgical Instrument Tracking for Intraoperative Vitrectomy Guidance Using Deep Learning and Computer Vision. University of Illinois at Chicago
- **RONNEBERGER, O ; FISCHER, P. ; BROX, T.** *U-net: Convolutional networks for biomedical image segmentation,* 2015

- **VINCENT, L. ; SOILLE, P.** Watersheds in digital spaces: an efficient algorithm based on immersion simulations. *IEEE Transactions on Pattern Analysis & Machine Intelligence,* 1991, vol. 6, 583-598
- **BEUCHER, S.** Use of watersheds in contour detection. *Proceedings of the International Workshop on Image Processing. CCETT,* 1979
- **MEYER, F. ; BEUCHER, S.** Morphological segmentation. *Journal of visual communication and image representation,* 1990, vol. 1 (1), 21-46
- A fast 2d otsu thresholding algorithm based on improved histogram. **ZHU, N. ; WANG, G. ; YANG, G. ; DAI, W.** Pattern Recognition, 2009. CCPR 2009. Chinese Conference on. IEEE, 2009, 1-5